(19) Europäisches Patentamt
European Patent Office
Office européen des brevets

(11) Publication number: 0 050 800 B2

# (12) NEW EUROPEAN PATENT SPECIFICATION

(45) Date of publication of the new patent specification: **07.06.95**

(51) Int. Cl.6: **C07K 5/06**, A61K 38/55

(21) Application number: **81108348.4**

(22) Date of filing: **15.10.81**

The file contains technical information submitted after the application was filed and not included in this specification

## (54) Carboxyalkyl dipeptides, processes for their production and pharmaceutical compositions containing them.

(30) Priority: **23.10.80 US 199886**
**28.04.81 US 258484**

(43) Date of publication of application:
**05.05.82 Bulletin 82/18**

(45) Publication of the grant of the patent:
**18.06.86 Bulletin 86/25**

(45) Mention of the opposition decision:
**07.06.95 Bulletin 95/23**

(84) Designated Contracting States:
**AT BE CH DE FR GB IT LI LU NL SE**

(56) References cited:
**EP-A- 0 012 401** **EP-A- 0 012 845**
**EP-A- 0 037 231** **EP-A- 0 048 159**
**EP-A- 0 049 658** **EP-A- 0 052 991**
**EP-A- 0 054 862** **FR-A- 7 931 132**
**US-A- 3 514 465** **US-A- 4 105 776**

**A Burger, "Medicinal Chemistry, Wiley Interscience, NY, 3rd ed. (1970), p.75-76**

**A Lespagnol, Chimie des Medicaments, Tome 1, Paris (1974) p.6-13, p.290**

(73) Proprietor: **SCHERING CORPORATION**
**2000 Galloping Hill Road**
**Kenilworth**
**New Jersey 07033 (US)**

(72) Inventor: **Neustadt, Bernard R.**
**24 Brook Place**
**West Orange**
**New Jersey 07052 (US)**
Inventor: **Gold, Elijah H.**
**10 Roosevelt Avenue**
**West Orange**
**New Jersey 07052 (US)**
Inventor: **Smith, Elizabeth M.**
**166 Grove Avenue**
**Verona**
**New Jersey 07004 (US)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**



Rank Xerox (UK) Business Services
(3.10/3.09/3.3.3)

D W Cushman et al.:"Federation Proceedings vol.38, No.13 (1979), p.2778-2782

D W Cushman et al, Biochem Pharmacol. 20, p.1637-1648

E C Taylor et al., Heterocycles vol.25. p.343-345

E C Taylor et al. J Org Chem, vol. 38, No 16 p.2817-2821

J Org Chem., vol. 22, 1957, p.1261-1263

## Description

The present invention relates to carboxyalkyl dipeptides which are useful as inhibitors of angiotensin-converting enzyme and as antihypertensive agents.

Carboxyalkyl dipeptides which are useful as inhibitors of angiotensin-converting enzyme and as anti-hypertensive agents are known from the published European patent application No. 12401.

The compounds of the present invention are compounds of the formula

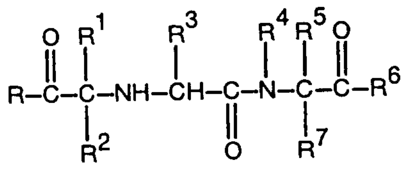

I

and the pharmaceutically acceptable salts thereof, wherein R and $R^6$ are the same or different and are hydroxy, lower alkoxy, lower alkenyloxy, dilower alkylamino lower alkoxy (e.g. dimethylaminoethoxy), acylamino lower alkoxy (e.g. acetylaminoethoxy), acyloxy lower alkoxy (e.g. pivaloyloxyethoxy), aryloxy (e.g. phenoxy), arylloweralkoxy (e.g. benzyloxy), amino, lower alkylamino, dilower alkylamino, hydroxyamino, aryllower alkylamino (e.g. benzylamino), or substituted aryloxy or substituted aryllower alkoxy wherein the substituent is methyl, halo or methoxy; $R^1$ is hydrogen, alkyl of from 1 to 10 carbon atoms, including branched and cyclic and unsaturated (e.g. allyl) alkyl groups, substituted lower alkyl wherein the substituent is hydroxy, lower alkoxy, aryloxy, (e.g. phenoxy), substituted aryloxy, heteroaryloxy, substituted heteroaryloxy, amino, lower alkylamino, diloweralkylamino, acylamino, arylamino, substituted arylamino, guanidino, imidazolyl, indolyl, lower alkylthio, arylthio (e.g. phenylthio), substituted arylthio, carboxy, carbamoyl, lower alkoxycarbonyl, aryl (e.g. phenyl or naphthyl), substituted aryl, aralkyloxy, substituted aralkyloxy, aralkylthio, or substituted aralkylthio, wherein the aryl or heteroaryl portion of said substituted aryloxy, heteroaryloxy, arylamino, arylthio, aryl, aralkyloxy or aralkylthio groups is substituted with a group selected from halo, loweralkyl, hydroxy, lower alkoxy, amino, aminomethyl, carboxyl, cyano and sulfamoyl; $R^2$ and $R^7$ are the same or different and are hydrogen or lower alkyl; $R^3$ is hydrogen, lower alkyl, phenyl lower ally, aminomethylphenyl lower alkyl, hydroxyphenyl lower alkyl, hydroxy lower alkyl, acylamino lower ally (e.g. benzoylamino lower alkyl or acetylamino lower alkyl), amino lower alkyl, dimethylamino lower alkyl, guanadino lower alkyl, imidazolyl lower alkyl, indolyl lower alkyl, or lower alkylthio lower alkyl; $R^4$ and $R^5$ taken together form a group represented Q or U, wherein;

Q is

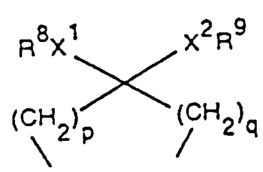

wherein $X^1$ and $X^2$ independent of each other are O, S or $CH_2$, $R^8$ and $R^9$ taken together form a bridge W, wherein W is an alkylene or substituted alkylene bridge having 2 or 3 carbon atoms, said substituted alkylene bridge having one or two substituents selected from lower alkyl, aryl, and aryl lower alkyl groups. p is 0, 1 or 2, q is 0, 1 or 2, with the proviso that the sum of p and q must be 1, 2 or 3, with the proviso that if p is 0 then $X^1$ and $X^2$ must be methylene;

U is

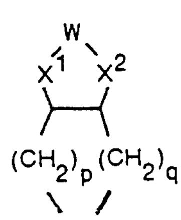

,wherein W is a methylene bridge or a substituted methylene bridge when at least one of $X^1$ and $X^2$ is methylene, or W is an alkylene or substituted alkylene bridge having 2 or 3 carbon atoms, said substituted methylene bridge or said substituted alkylene bridge having one or two substituents selected from lower alkyl, aryl and aryl lower alkyl groups, (except that W may also be a methylene bridge when $X^1$ and $X^2$ are oxygen or sulfur), $X^1$ and $X^2$ are as defined above, p is 0, 1 or 2, q is 0, 1 or 2, with the proviso that the sum of p and q is 1 or 2, and with the proviso that if p is 0, $X^1$ must be $CH_2$, excluding compounds wherein U is

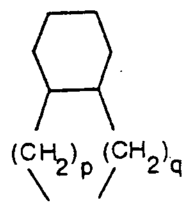

wherein p is zero or 1 and q is 1;

As will be seen from the above descriptions of the compounds of the present invention, when $R^4$ and $R^5$ form a group Q or U, these groups, taken together with the nitrogen' to which $R^4$ is attached and the carbon to which $R^5$ is attached, form various ring systems. Included among these ring systems are the following:

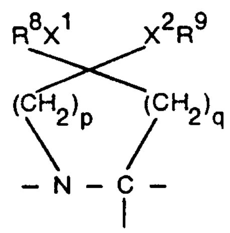

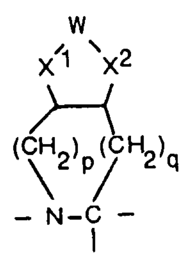

$R^8$ and $R^9$ in the group Q form a bridge W, as described above, whereby the following ring system is formed:

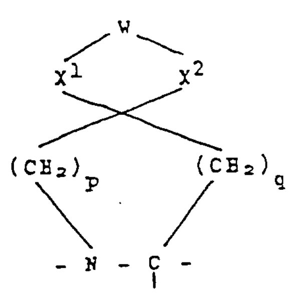

Each of the rings in the structures shown above will have at least four members. The values of p and q, and the chosen definition of W in the rings drawn above will determine whether any of the aforesaid rings will have 5 or 6 or more members.

Another embodiment of the present invention comprises compounds of the formula

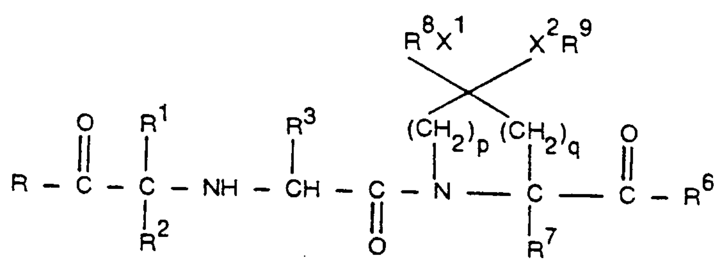

III

wherein $R^8$, $R^9$, $X^1$, $X^2$, p and q are as defined above for the group Q and wherein R, $R^1$, $R^2$, $R^3$, $R^6$ and $R^7$ are as defined above.

Non-limiting examples of compounds of the formula *III* are

compounds wherein $X^1$ and $X^2$ are methylene, $R^8$ and $R^9$ taken together form the bridge W, and p, q, and W are as defined above, especially wherein W is an ethylene bridge; wherein p and q are each 1, or wherein p is 0 and q is 2;

compounds, wherein $X^1$ and $X^2$ are S, and $R^8$, $R^9$, p and q are as defined above, especially compounds wherein $R^8$ and $R^9$ taken together form an ethylene bridge and p and q are as defined above, preferably are each 1 or p is 1 and q is 2.

Another embodiment of the present invention comprises compounds of the formula

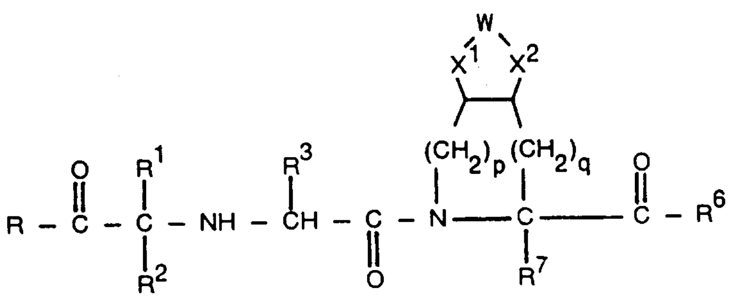

VI

wherein $X^1$, $X^2$, W, p and q are as defined above for the group U and R, $R^1$, $R^2$, $R^3$, $R^6$ and $R^7$ are as defined above.

Non-limiting examples of compounds of the formula *VI* are

compounds, wherein $X^1$ and $X^2$ are methylene and W is methylene and p and q are as defined above, especially wherein p is 0 and q is 1 and wherein p is 0 and q is 2;

compounds, wherein $X^1$ and $X^2$ are methylene and W is ethylene and p and q are as defined above, especially wherein p is 1 and q is 0 and wherein p is 0 and q is 2;

compounds wherein $X^1$ and $X^2$ are methylene and W is trimethylene and p and q are as defined above, especially wherein p is 0 and q is 1;

compounds wherein $X^1$ and $X^2$ are methylene and W is methylene, ethylene or trimethylene substituted with one or two lower alkyl groups and p and q are as defined above, especially wherein p is 0 and q is 1;

compounds, wherein $X^1$ and $X^2$ are O, W is methylene and p and q are as defined above.

In the above described compounds R, $R^1$, $R^2$ $R^3$, $R^6$ arid $R^7$ can be exemplified as follows:

$R^1$ is substituted lower alkyl, wherein the substituent is unsubstituted or lower-alkyl-substituted aryl, aralkyloxy or aralkylthio, in particular $R^1$ is substituted lower alkyl, wherein the substituent is aralkyloxy or aralkylthio, especially benzyloxy or benzylthio;

R and $R^6$ are the same or different and are hydroxy or lower alkoxy, especially R is hydroxy, methoxy or ethoxy and $R^6$ is hydroxy, ethoxy or benzyloxy;

$R^2$ and $R^7$ are hydrogen;

$R^3$ is hydrogen, lower alkyl or phenyl lower alkyl, especially hydrogen, methyl or benzyl.

Preferred subclasses of the present invention are aminoacyl-azabicycloalkane carboxylic acids, more preferably alanyl azabicycloalkane carboxylic acids and most preferably N-(alkoxycarbonyl alkylalanyl)-azabicycloalkane carboxylic acids.

Other preferred subclasses of the present invention are N-(alkoxycarbonyl-aralkyloxyalkyl) dipeptides and N-alkoxycarbonyl-aralkylthio-alkyl) dipeptides. Particularly preferred are N-(alkoxycarbonyl-aralkylthioalkyl)-alanyl aminoacids, and most preferred are N-(alkoxycarbonyl-aralkylthioalkyl)-alanyl azacycloalkane carboxylic acids and the corresponding azabicycloalkane carboxylic acids.

The aforementioned compounds of the formula *I*, as defined above, include all possible stereoisomers. Acyl includes —OC—$R^{12}$, wherein $R^{12}$ is lower alkyl, lower alkenyl or aryl. The lower alkyl, lower alkenyl or lower alkynyl groups except where noted otherwise are represented by any of the variables including straight and branched chain hydrocarbon radicals from one to six carbon atoms, for example, methyl, ethyl, propyl, isopropyl, butyl, isobutyl, t-butyl, pentyl, isopentyl, hexyl or vinyl, allyl, butenyl and the like. Cycloalkyl groups (containing 3 to 8 carbon atoms) include bridged and non-bridged groups. The aralkyl groups represented by any of the above variables have from one to four carbon atoms in the alkyl portion thereof and include for example, benzyl, p-methoxybenzyl and the like. Halo means chloro, bromo, iodo or fluoro. Aryl where it appears in any of the radicals except where noted otherwise represents phenyl or naphthyl. Heteroaryl groups where they appear include for example pyridyl, thienyl, furyl, indolyl, benzthienyl, imidazolyl and thiazolyl. The $R_1$ and $R_3$ substituted lower alkyl moieties are exemplified by groups such as

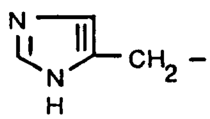
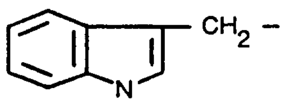
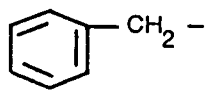

HO—$CH_2$—, HS—$CH_2$—, $H_2N$—$(CH_2)_4$—, $CH_3$—S—$(CH_2)_2$—, $H_2N$—$(CH_2)_3$—,

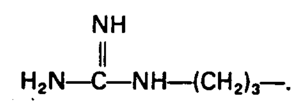

In the compounds of the formula *I*, the carbon atoms to which $R^1$, $R^3$ and $R^5$ are attached may be *asymmetric*. The compounds accordingly exist in diastereoisomeric forms or in mixtures thereof.

In general, the aminoacid part-structures, i.e.,

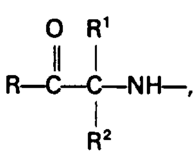
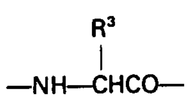
and
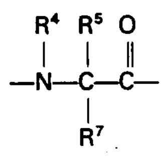

of Formula *I* are preferred in the configuration most similar to that of natural L-amino acids. Usually, natural L-amino acids are assigned the *S*-configuration. A notable exception is the natural amino acid L-cysteine which is assigned the *R*-configuration.

The compounds of the present invention can be produced by one or more of the methods and subroutes depicted in the following equations. Reactive groups not involved in the condensations described below such as amino, carboxy, mercapto, etc., may be protected by methods standard in peptide chemistry prior to the coupling reactions and subsequently deprotected to obtain the desired products. In other words, in the formula of the following description of the processes R, $R^1$, $R^2$, $R^3$, $R^4$, $R^5$, $R^6$ and $R^7$ are as defined above for Formula I, including suitable protection.

A. For the preparation of compounds of formula I, wherein $R^2$ is hydrogen a ketocompound (XIII) is condensed with a dipeptide (XIV) under reduction.

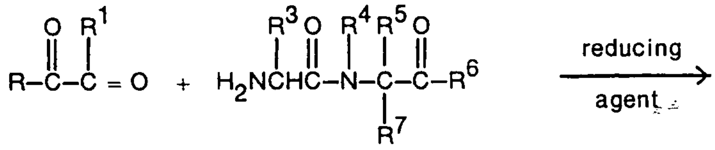


I

XIII XIV

The ketocompound (XIII) can be condensed with the dipeptide (*XIV*) in aqueous solution, optimally near neutrality, or in a suitable organic solvent (for example, $CH_3OH$) in the presence of a reducing agent such as for example sodium cyanoborohydride to give directly the desired compound I. Alternatively, the intermediate Schiff base, enamine, or aminol may be catalytically reduced to yield product *I*, for example, by hydrogen in the presence of palladium on carbon (e.g. 10% palladium on carbon) or of Raney nickel. The ratio of diasteriomeric products formed may be altered by choice of catalyst.

B. Alkylation of a dipeptide (XIV) by means of a compound *XXII*

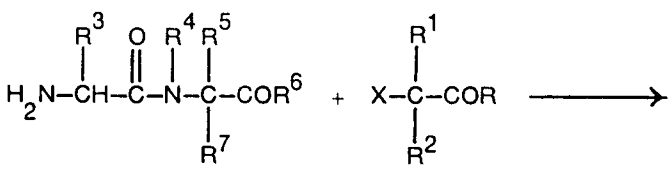

I

XIV XXII

wherein X is chloro, bromo, iodo, alkanesulfonyloxy or arenesulfonyloxy. The reaction can be carried out under basic conditions in water or in an organic solvent.

C. Condensation of an amino compound (XVIII) with a keto-compound (XIX)

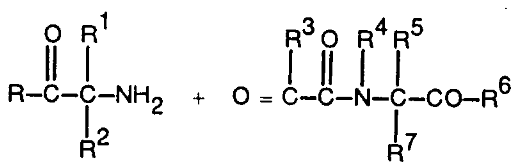

I

XVIII XIX

under the conditions described for process A.

D. Alkylation of an aminocompound (*XVIII*) by means of a compound XXIII.

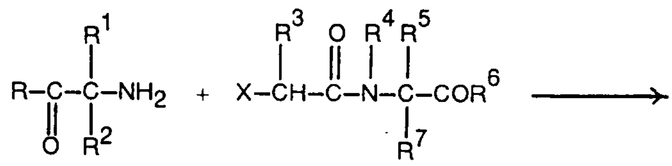

I

XVIII XXIII

wherein X is chloro, bromo, iodo, alkanesulfonyloxy or arenesulfonyloxy. The reaction can be carried out under the conditions described for process B.

E. Condensation of an aminoacid XXI with an aminoacid XVII

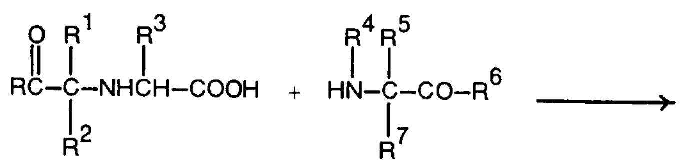

I

XXI XVII

This reaction is well known from peptide chemistry. The reaction can be carried out in the presence of a condensing agent such as for example dicyclohexylcarbodiumide (DCC), diphenylphosphoryl azide (DPPA) and N,N-disuccinimidyl carbonate in $CH_3CN$. While, as mentioned above, reactive groups (in R, $R^1$, $R^3$, $R^4$, $R^5$ and $R^6$) are protected before the coupling reaction is carried out, the amino group of compound XVII can be activated, e.g. by means of tetraethyldiphosphit and/or the carboxy group of compound XXI can be activated via the intermediacy of active esters such as that derived from 1-hydroxybenzotriazole, its mixed anhydride (derived from a chlorocarbonic acid ester), its azide or dicyclohexylcarbodiimide.

The starting compounds in this reaction are known compounds and/or can be prepared according to known methods.

The compound of formula XXI, wherein $R^2$ is hydrogen can for example be prepared by reacting a keto compound XIII with an amino acid XV

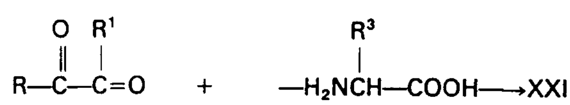

according to the conditions described in process A. Alternatively compound XXI can be prepared by condensing XVIII with a ketoacid XX

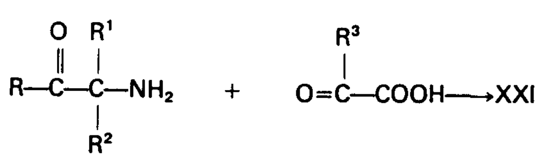

*XVIII* *XX*

or by condensing XV and XXII or XVIII and XXIV

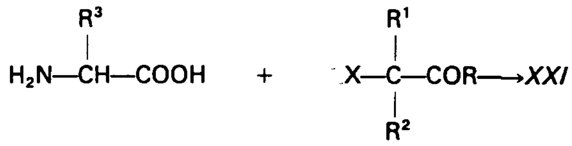

*XV* *XXII*

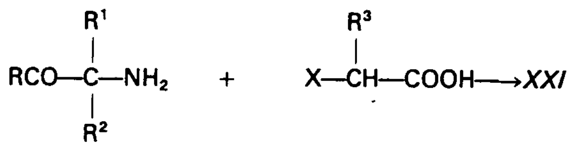

*XVIII* *XXIV*

under the conditions described for process B above (X being as defined in process B).

It is evident that a compound of formula I obtained by any one of processes A to E can be transformed into another compound of formula I by methods known in the art.

The above processes are followed by setting free protected groups by known methods. Protected carboxy groups, e.g. when R and $R^6$ are for example alkoxy (methoxy, ethoxy, tert.butyloxy), nitrobenzyloxy or benzyloxy, are set free by hydrolysis or hydrogenation. (Reductive cleavage of a benzyl ester / (where $R^6$ is benzyloxy and R is alkoxy) will yield compounds of formula *I* wherein R is alkoxy and $R^6$ is hydroxy, and where $R^6$ is alkoxy and R is benzyloxy, will yield compounds of formula *I* wherein R is hydroxy and $R^6$ is alkoxy). Hydrolysis can be carried out under acidic conditions (using e.g. a halogen hydracid or trifluoroacetic acid), under basic conditions or by means of photochemical hydrolysis.

The amino group(s) can be protected by protecting groups such as for example formyl, t-butoxycarbonyl, carbobenzyloxy, triphenylmethyl and nitrophenylsulfenyl. These groups can be removed under acidic conditions, e.g. by means of a halogenhydroacid and/or trifluoroacetic acid.

In the special case of $R^1$ bearing an alpha-amino substituent, the carbonyl and amino groups can be conveniently protected as a beta-lactam function. This kind of protection can be removed by known methods, e.g. as described above for the hydrolysis.

In the compounds of formula *I*, the carbon atoms to which $R^1$, $R^3$ and $R^5$ are attached may be asymmetric. The compounds accordingly exist in diastereoisomeric forms or in mixtures thereof. The above described syntheses can utilize racemates, enantiomers or diastereomers as starting materials. Enantiomeric intermediates may be obtained by resolution methods known in the art. When diastereomeric products result from the synthetic procedures, the diastereomeric products can be separated by conventional chromatographic or fractional crystallization methods.

The compounds of this invention form salts with various inorganic and organic acids and bases which are also within the scope of the invention. Such salts include ammonium salts, alkali metal salts like sodium and potassium salts (which are preferred), alkaline earth metal salts like the calcium and magnesium salts, salts with organic bases e.g., dicyclohexylamine salts, N-methyl-D-glucamine, salts with amino acids like arginine, lysine and the like. Also, salts with organic and inorganic acids may be prepared, e.g., HCl, HBr, $H_2SO_4$, $H_3PO_4$, methanesulfonic acid, toluenesulfonic acid, maleic acid, fumaric acid and camphorsulfonic acid. The non-toxic physiologically acceptable salts are preferred, although other salts are also useful, e.g., in isolating or purifying the product.

The salts may be formed by conventional means, as by reacting the free acid or free base forms of the product with one or more equivalents of the appropriate base or acid in a solvent or medium in which the salt is insoluble, or in a solvent such as water which is then removed *in vacuo* or by freeze-drying or by exchanging the cations of an existing salt for another cation on a suitable ion exchange resin.

The compounds of this invention have useful pharmacological properties. They are useful in the treatment of high blood pressure.

The compounds of the present invention can be combined with pharmaceutical carriers and administered in a variety of well known pharmaceutical forms suitable for oral or parenteral administration to provide compositions useful in the treatment of cardiovascular disorders and particularly mammalian

hypertension.

The dosage of the compounds of this invention will typically be in the range of about 0.01 to about 30 mg/kg, preferably about 0.1 to about 10 mg/kg, of mammalia weight, administered in single or divided doses. The exact dose to be administered is dependent upon where the particular compound lies within the above quoted range, as well as upon the age, weight and condition of the individual.

Generally, in treating humans, the compounds of this invention may be administered to patients in need of such treatment in a dosage range of 5 to 500 mg per patient generally given several times, thus giving a total daily dose of from 5 to 2000 mg per day. Also, the compounds of this invention may be given in combination with diuretics or other antihypertensives. Typically, these are combinations whose individual dosages range from one-fifth of the minimally recommended clinical dosages to the maximum recommended levels for the entities when they are given singly. Examples of such diuretics or other antihypertensives are hydrochlorothiazide, ethacrynic acid, amiloride, furosemide, propanolol, timolol, methyldopa and chlorothiazide.

The composition containing the compounds of this invention will preferably contain from about 5 to about 250 mg of the active compound per dosage unit. These compositions are most preferably administered orally. Typical formulations for oral administration are those such as tablets, capsules, syrups, elixirs or suspensions. Typical injectable formulations include solutions and suspensions.

Typical acceptable pharmaceutical carriers for use in the formulations described above are exemplified by: sugars such as lactose, sucrose, mannitol and sorbitol; starches such as corn starch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate, calcium sulfate, polyvinylpyrrolidone, polyvinyl alcohol; stearic acid; alkaline earth metal stearates such as magnesium stearate and calcium stearate, stearic acid; vegetable oils such as peanut oil, cottonseed oil, sesame oil, olive oil and corn oil; nonionic, cationic and anionic surfactants; ethylene glycol polymers; beta-cyclodextrin; fatty alcohols and hydrolyzed cereal solids; as well as other non-toxic compatible fillers, binders, disintegrants, buffers, preservatives, antioxidants, lubricants, flavoring agents, and the like commonly used in pharmaceutical formulations.

The following examples illustrate the preparation of the compounds of the present invention. The diasteromers prepared as set forth below may be isolated by column chromatography or by fractional crystallization. Preparations 1 to 5 illustrate details of suitable analogy processes. The products of preparations 1 to 5 are excluded from the claimed subject matter.

In the preparations 1 to 5, octahydroindole-2(S)-carboxylic acid refers to *cis,syn*-octahydroindole-2(S)-carboxylic acid, also named 3a(S), 7a(S)-octahydroindole-2(S)-carboxylic acid.

Preparation 1

1-[N-(1-Carbomethoxy-3-phenylpropyl)-(S)-alanyl]octahydroindole-2(S)-carboxylic acid

A. Dissolve 27.0 g of ethyl indole-2-carboxylate in 250 ml of trifluoroacetic acid. Add 2.05 g of platinium oxide, hydrogenate the mixture at 50 lb/in$^2$ at room temperature. Filter the mixture and concentrate the filtrate *in vacuo* to give a residue. Suspend the residue in ether and treat with cold dilute sodium hydroxide solution. Dry the organic layer dried over magnesium sulfate and concentrate it to give ethyl octahydroindole-2-carboxylate, a pale yellow oil. The oil should immediately be used in the following step.

B. To a solution of 10.0g of ethyl octahydroindole-2-carboxylate (prepared in as shown in paragraph A of this preparation) in 400 ml of ethyl acetate add 17.0 g of N-benzyl-oxycarbonyl-(S)-alanine, N-hydroxysuccinimide ester. Stir the reaction mixture at room temperature for 20 hours and concentrate it *in vacuo*. Place the residue on a column of silica gel (3000 g, particle size 0.25—0.074 mm, (60—200 mesh)) and elute with chloroform:ethyl acetate 10:1 to give 1-[N-benzyloxycarbonyl-(S)-alanyl]-octahydroindole-2(R)-carboxylic acid ethyl ester, a colorless oil $[\alpha]_D^{26}$ +22.0° (ethanol) and 1-[N-benzyloxycarbonyl-(S)-alanyl]octahydroindole-2(S)-carboxylic acid ethyl ester, a colorless oil $[\alpha]_D^{26}$ -96.4° (ethanol).

C. To a solution of 3.22 g of 1-[N-benzyloxycarbonyl-(S)-alanyl]octahydroindole-2(S)-carboxylic acid, ethyl ester in 150 ml of methanol, add 20 ml of 2.5 N sodium hydroxide and stir the mixture at room temperature for 18 hours. Concentrate the mixture under nitrogen, dilute the residue with ice-water and then make the mixture acidic with concentrated hydrochloric acid. Extract the aqueous solution with ethyl acetate and dry the organic phase over magnesium sulfate. Concentrate the organic phase and place it on a column of silica gel (500 g., particle size 0.25—0.074 mm, (60—200 mesh)). Elute with chloroform:glacial acetic acid 9:1 to give 1-[N-benzyloxycarbonyl-(S)-alanyl]octahydroindole-2(S)-carboxylic acid, a white solid $[\alpha]_D^{26}$ -62.1° (ethanol), m.p. 58.60°.

D. Dissolve 1.70 g of 1-[N-Benzyloxycarbonyl-(S)-alanyl]octahydroindole-2(S)-carboxylic acid in 100 ml of methanol. Add 0.40 g 10% palladium-on-charcoal and hydrogenate the mixture at atmospheric pressure. Filter the mixture and concentrate *in vacuo* to give 1-[(S)-alanyl]octahydroindole-2(S)-carboxylic acid, a white solid $[\alpha]_D^{26}$ -18.5° (ethanol), m.p. 163—165°.

E. Dissolve 1-[(S)-alanyl]octahydroindole-2(S)-carboxylic acid (prepared in paragraph D of this preparation) in 100 ml of absolute methanol. Add 1.10 g 2-oxo-4-phenylbutyric acid, ethyl ester and 20 ml of 3 Angstrom molecular sieve pellets, and stir the resulting mixture at room temperature for eighteen hours. Filter the reaction mixture and treat the filtrate with 0.68 g sodium cyanoborohydride at room temperature for two hours. Concentrate the mixture under nitrogen and dilute the oil with dilute hydrochloric acid and stir at room temperature for one hour. Absorb the aqueous solution on 200 ml of an XAD—2 (Rohm & Haas Co. resin). Elute the resin with 2000 ml of water and then with 2000 ml of methanol. Concentrate the methanol solution and place the residue on a column of silica gel (400 g, 60—200 mesh) and elute with chloroform:isopropanol:7% ammonium hydroxide 1:1:1 (organic layer) to give 1[N(1-ethoxycarbonyl-3-phenylpropyl)-(S)-alanyl)octahydroindole-2(S)-carboxylic acid, a white solid $[\alpha]_D^{26}$ -45.2° (ethanol), m.p. 71—73°.

Preparation 2

1-[N-(1-carboxy-3-phenylpropyl)-(S)-alanyl]octahydroindole-2(S)-carboxylic acid

To a solution of 1-[N-(1-carbomethoxy-3-phenylpropyl)-(S)-alanyl]octahydroindole-2(S)-carboxylic acid (prepared as described in preparation 1) in methanol, add 2.5 N sodium hydroxide. After three hours, concentrate the reaction mixture and absorb it on an XAD—2 resin column and elute with water and then with methanol. Concentrate the methanol eluant to give a residue and absorb this residue on a silica gel column and elute with chloroform:methanol:14% ammonium hydroxide 1:1:1. Concentrate the desired eluant fractions to obtain the title compound.

Preparation 3

1-[N-(1(S)-carboethoxy-3-phenylpropyl)-(S)-alanyl]-octahydroindole-2(S)-carboxylic acid

Following the procedure of Preparation 1, but modifying step E to use ethanol as reaction solvent, prepare 1-[N-(1(R,S)-carboethoxy-3-phenylpropyl)-(S)-alanyl-octahydroindole-2(S)-carboxylic acid. Chromatograph this material on an RP—8 reversed-phase column using acetonitrile:0.2N $NH_4OAc$ 40:60 (pH 8.6) as eluent to obtain the title compound as a solid $[\alpha]_D^{26}$ = -45.3° (ethanol).

Preparation 4

1-{N-[1(S)-carboethoxy-3-phenylpropyl]-(S)-alanyl}-3a(S),7a(S)-octahydroindole-2(S)-carboxylic acid

A. To a 100 ml three-neck flask, equipped with a thermometer, dropping funnel, magnetic stirrer and ice bath, 23 ml (0.21 mole) of benzyl alcohol was added and cooled to 0°C under $N_2$. $SOCl_2$ (5.95 g, 3.7 ml, 0.05 mole) was added dropwise over 15 min, maintaining the temperature at *ca.* 0°C. *Cis,syn*-perhydroindole-2(S)-carboxylic acid (ca. 0.21 mole) was added and the mixture was stirred at *ca.* 0°C for 1 hour and then for 24 hours at room temperature. The resulting mixture was poured into 500 ml of ether, stirred under $N_2$ for 1 hour and then allowed to stand under $N_2$ until it was no longer cloudy. The mixture was decanted and the oily precipitate was washed with 25 ml of ether, then slurried in 200 ml of ether followed by addition of 1N NaOH to pH 8—9. The mixture was stirred for 5 minutes, and the organic layer was then washed with brine, dried over $MgSO_4$, filtered and evaporated in vacuo at room temperature to give 2-(S)-benzyloxycarbonyl-*cis,syn*-octahydroindole as a colorless oil (t)c (ether) one spot, $R_f$ ~0.31.

B. To a 5 l flask equipped with a magnetic stirrer, dropping funnel and $N_2$ inlet tube, was added a solution of 190 g (0.92 mole) of ethyl 2-oxo-4-phenyl butanoate, and 258 g (0.734 mole) of S-alanine benzylester p-toluenesulfonate in 1.4 l of EtOH. This yellow solution was stirred for 2 hours under $N_2$. A solution of 17.7 g (0.282 mole) of $NaBH_3CN$ in 550 ml of EtOH was then added, with stirring, over 90 minutes. The solution was stirred overnight and concentrated to dryness in vacuo at room temperature.

The residue was partitioned between 500 ml of $H_2O$ and 2 l of ether and the ether layer was dried over $MgSO_4$ and filtered. To this solution, 1.3M ethereal HCl was added to pH 4. The ether and excess HCl were then removed in vacuo at room temperature. The residue was slurried in 250 ml of ether and diluted with 750 ml of hexane. The supernatent was decanted from the resulting precipitate. The precipitate was washed with two 300 ml portions of ether as above. The residue was then triturated with 300 ml of ether and filtered under $N_2$ to give a white solid. This was slurried in ether and made basic with saturated aqueous $NaHCO_3$. The organic layer was dried over $MgSO_4$, filtered and concentrated in vacuo at room temperature to give a yellow oil. This oil was dissolved in 510 ml of EtOAc, to which was added a hot solution of 40.5 g of maleic acid in 895 ml of EtOAc. After cooling to room temperature the resulting precipitate was filtered and recrystallized from EtOAc to give hemimaleate as a white solid, mp 127—128 °C, $[\alpha]_D^{26}$ 0 ° (c = 1 % $H_2O$) [tlc (cyclohexane: EtOAc — 85:15) shows one spot $R_f$ ~0.30) after neutralization]. 7.0 g (.015 mole) of the so obtained product was slurried in EtOAc and made basic with saturated aqueous $NaHCO_3$. The organic layer was washed with brine, dried over $MgSO_4$, filtered and concentrated in vacuo at room temperature to give the product as a colorless oil. This was dissolved in 100 ml of EtOH containing 0.7 g of 10% Pd/C. The mixture was hydrogenated in a Parr shaker under pressure for 2 hours. After filtration, the solvent was removed in vacuo at room temperature to afford 4.0 g (82%) of N-(1-(S)-ethoxycarbonyl-3-phenylpropyl)-(S)-alanine as a white solid, mp 147—148 °C, $[\alpha]_D^{26}$ + 24.8 ° (c = 1% MeOH) [$R_f$ ~0.1 (EtOAc:MeOH:HOAc — 100:1:1)].

C. To a 50 ml three-neck flask equipped with a dropping funnel, thermometer, magnetic stirrer and an ice bath, a solution of 0.23 g of 2-(S)-benzyloxycarbonyl-*cis-syn*-octahydroindole and 0.25 g (0.0009 mole) of N-(1-(S)-ethoxycarbonyl-3-phenylpropyl)-(S)-alanine in 5.0 ml of DMF was added and cooled under $N_2$ to 0 °C. With stirring, 0.14 g (0.00135 mole) of N-methylmorpholine was added, followed by dropwise addition (5 min) of a solution of 0.25 g (0.0009 mole) of diphenylphosphorylazide in 5 ml of DMF, (temperature of mixture maintained at 0—10 °C). The solution was stirred at this temperature for 1 hour and then at room temperature overnight. Saturated aqueous $NaHCO_3$ was added to the light yellow solution to pH 8. It was then extracted with ether, dried over $MgSO_4$, filtered and evaporated in vacuo at room temperature to give 0.43 g of crude product as a yellow oil. This was preadsorbed on 1 g of coarse (particle size 0.25—0.074 mm) ((60—200 mesh) silica gel and then gravity filtered through 40 g of coarse silica gel (60—200 mesh) with *ca*. 300 ml of ether. 1-[N-[1(S)-ethoxycarbonyl-3-phenylpropyl]-(S)-alanyl]-*cis-syn*-octahydroindole-2-(S)-carboxylic acid benzyl ester was obtained as a colorless oil.

D. 0.20 g (0.00038 mole) of the so obtained product was dissolved in 50 ml of EtOH containingg 0.04 g of 10% Pd/C. The mixture was hydrogenated in a Parr shaker at 60 psi (rt) for 2 hours. The mixture was filtered and the filtrate was evaporated in vacuo at room temperature to yield the title compound as a colorless oil.

Preparation 5

1-{N-[1(S)-carboethoxy-3-phenylpropyl]-(S)-alanyl}-3a(S),7a(S)-octahydroindole-2(S)-carboxylic acid

N-(1-(S)-ethoxycarbonyl-3-phenylpropyl)-(S)-alanine (400 mg, 0.0014 mole) (obtainable according to preparation 4 step B) was dissolved in 40 ml of dry $CH_3CN$ containing 0.417 g (0.0014 mole) of N,N-disuccinimidyl carbonate. Dry pyridine (0.10 ml, 0.0014 mole) was added and the solution was stirred at room temperature under $N_2$ overnight. *Cis*,*syn*-perhydroindole-2(S)-carboxylic acid (0.0014 mole) and 0.25 ml $Et_3N$ were then added and the mixture was stirred overnight. The solvent was evaporated in vacuo at room temperature to give a yellow gum. This was chromatographed on 100 g silica gel (tlc grade — EtOAc:MeOH:HOAc — 100:1:1) to afford the title compound as its acetate salt (gum).

Example 1

1-[N-(1-carboethoxy-3-phenylpropyl)-(S)-alanyl]decahydroquinoline-2(S)-carboxylic acid

Use ethyl decahydroquinoline-2-carboxylate (prepared by hydrogenation of quinoline-2-carboxylic acid in glacial acetic acid with platinum oxide followed by esterification in ethanol) in place of ethyl octahydroindole-2-carboxylate in Preparation 1B. Continue the sequence of reactions described in Preparation 1) to obtain the title compound.

Example 2

1-[N-(1-carboxy-3-phenylpropyl)-(S)-alanyl]decahydroquinoline-2(S)-carboxylic acid

As described in Preparation 2, treat 1-[N-(1-carboethoxy-3-phenylpropyl)-(S)-alanyl]decahydroquinoline-2-carboxylic acid (prepared as described in Example 1) with sodium hydroxide to obtain the title compound.

Example 3

2-[N-(1-carboethoxy-3-phenylpropyl)-(S)-alanyl]octahydroisoihdole-1(S)-carboxylic acid

A. Heat *cis*-octahydroisoindole (prepared by reduction or *cis*-hexahydrophthalimide in tetrahydrofuran with lithium aluminum hydride) and mercuric acetate in 10% aqueous acetic acid under reflux for twenty hours to give *cis*-hexahydro-$\Delta^1$-isoindole. Dissolve this compound in water and treat with potassium cyanide followed by 2N hydrochloric acid at 0° for two hours and at room temperature for twenty hours to give 1-cyano-*cis*-octahydroisoindole. Heat this cyano compound in 6N hydrochloric acid under reflux for 6 hours followed by concentration of the reaction mixture and absorption of the residue on an XAD—2 resin column. Elute with methanol to obtain *cis*-octahydroisoindole-1-carboxylic acid.

B. Use ethyl *cis*-octahydroisoindole-1-carboxylate (prepared by esterification with ethanol of the acid prepared in paragraph A next above) in place of ethyl octahydroindole-2-carboxylate in Preparation 1B through 1E to give the title compound.

Example 4

2-[N-(1-carboxy-3-phenylpropyl)-(S)-alanyl]octahydroisoindole-1(S)-carboxylic acid

As described in Preparation 2 treat N-(1-carboethoxy-3-phenylpropyl)-(S)-alanyl]octahydroisoindole-1-(S)-carboxylic acid (prepared as described in Example 3) with sodium hydroxide to obtain the title compound.

Example 5

1-[N-(1-carboethoxy-3-phenylpropyl)-(S)-alanyl]octahydrocyclopenta[b]pyrrole-2(S)-carboxylic acid

A. Substitute octahydrocyclopenta[b]pyrrole (prepared by reduction of 2-ketooctahydrocyclopenta[b]-pyrrole in tetrahydrofuran with lithium aluminum hydride) for octahydroisoindole in Example 3A to obtain octahydrocyclopenta[b]pyrrole-2-carboxylic acid.

B. Use ethyl octahydrocyclopenta[b]pyrrole-2-carboxylate (prepared by esterification with the ethanol of the acid prepared as described in paragraph A) in place of ethyl octahydroindole-2-carboxylate in the procedure described in paragraphs B through E of Preparation 1 to give the title compound.

Example 6

1-(N-(1-carboxy-3-phenylpropyl)-(S)-alanyl]octahydrooyclopenta[b]pyrrole-2(S)-carboxylic acid

As described in Preparation 2, hydrolyze the ester (prepared as described in Example 5) with sodium hydroxide to obtain the title compound.

Example 7

5-[N-(1-carboethoxy-3-phenylpropyl)-(S)-alanyl]-2,2-dimethyl-octahydro-1,3-dioxolo[4,5-c]pyrrole-4(S)-carboxylic acid

Heat 1-benzyloxycarbonyl-3,4-dihydroxy-(S)-proline [preparable from reaction of 3,4-dihydroxy-(S)-proline in 2N sodium hydroxide with benzyl chloroformate in ether] with 2,2-dimethoxy propane in dimethylformamide and p-toluenesulfonic acid to obtain 5-benzyloxycarbonyl-2,2-dimethyloctahydro-1,3-dioxolo[4.5-c]-pyrrole-4(S)-carboxylic acid.

Hydrogenate this compound in methanol with palladium on carbon to give 2,2-dimethyloctahydro-1,3-dioxolo[4,5-c]pyrrole-4(S)-carboxylic acid. React this compound with N-benzyloxycarbonyl-(S)-alanine, N-hydroxysuccinimide ester as described in Preparation 1B—E to isolate the title compound.

Example 8

7-[N-(carboethoxy-3-phenylpropyl)-(S)-alanyl]-1,4-dithia-7-azaspiro[4.4]nonane-8(S)-carboxylic acid

A. Dissolve 7.0 g of 1-benzyloxycarbonyl-4-keto-(S)-proline methyl ester in 75 ml of glacial acetic acid. Add 0.7 g of *p*-toluenesulfonic acid and 2.8 g of 1,2-ethanedithiol and heat under reflux with stirring for eighteen hours. Add the reaction mixture to saturated sodium bicarbonate solution and extract with ethyl acetate. Dry the organic layer over magnesium sulfate and concentrate it. Place the residue on a column of silica gel (300 g, 60—200 mesh) and elute with hexane:ethyl acetate (1:1) to give 7-benzyloxycarbonyl-1,4-dithia-7-azaspiro[4.4]nonane-8(S)-carboxylic acid, methyl ester, a yellow oil having $[\alpha]_D^{26}$ -12.6° (dioxane).

B. Dissolve 3.0 g of 7-benzoyloxycarbonyl-1,4-dithia-7-azaspiro[4.4]nonane-8(S)-carboxylic acid, methyl ester in 20 ml of 20% hydrobromic acid in glacial acetic acid and stir the mixture dropwise to diethyl ether at 0—5°C to give 1,4-dithia-7-azaspiro[4.4]nonane-8(S)-carboxylic acid, methyl ester hydrobromide, a brown solid m.p. 156—158°.

C. Dissolve the 1,4-dithia-7-azaspiro[4.4]nonane-8(S)-carboxylic acid, methyl ester, hydrobromide from paragraph B in 0.1*N* NaOH and extract with ethyl acetate. Dry the organic layer over magnesium sulfate and concentrate *in vacuo* to give 1,4-dithia-7-azaspiro[4.4]nonane-8(S)-carboxylic acid, methyl ester (1.35 g). Dissolve the latter in 100 ml of ethyl acetate and treat with 2.07 g of N-benzyloxycarbonyl-(S)-alanine, *N*-hydroxysuccinimide ester. Stir the reaction mixture at room temperature for eighteen hours and concentrate *in vacuo*. Place the residue on a column of silica gel (300 g, 60—200 mesh) and elute with hexane:ethyl acetate 4:1 to obtain 7-[N-benzyloxycarbonyl-(S)-alanyl]-1,4-dithia-7-azaspiro[4.4]-nonane-8(S)-carboxylic acid, methyl ester, a yellow oil $[\alpha]_D^{26}$ -14.8° (ethanol).

D. Dissolve 1.05 g of 7-[N-benzyloxycarbonyl-(S)-alanyl]-1,4-dithia-7-azaspiro[4.4]nonane-8(S)-carboxylic acid, methyl ester in 100 ml of methanol. Add 10 ml of 2.5N sodium hydroxide and stir the mixture at room temperature for sixteen hours. Concentrate the mixture under nitrogen, dissolve the oil in 0.1 N sodium hydroxide and dilute with ice water. Extract the aqueous solution with ethyl acetate. Acidify the aqueous solution with concentrated hydrochloric acid and then extract with ethyl acetate. Dry the organic phase over magnesium sulfate and concentrate it. Place the residue on a column of silica gel (100 g, 60—200 mesh) and elute with chloroform:glacial acetic acid 19:1 to obtain 7-[N-benzyloxycarbonyl-(S)-alanyl]-1,4-dithia-7-azaspiro[4.4]nonane-8(S)-carboxylic acid, $[\alpha]_D^{26}$ -15.8° (ethanol).

E. Dissolve 1.4 g of 7-[N-benzyloxycarbonyl-(S)-alanyl)-1,4-dithia-7-azaspiro[4.4]nonane-8(S)-carboxylic acid in 20 ml of 20% hydrobromic acid in glacial acetic acid and stir the mixture at room temperature for 2 hours. Add the mixture dropwise to diethyl ether at 0—5°C to give 7-[(S)-alanyl]-1,4-dithia-7-azaspiro-[4.4]-nonane-8(S)-carboxylic acid hydrobromide which is used immediately in the process described in paragraph F below.

F. Dissolve the 7-[(S)-alanyl]-1,4-dithia-7-azaspiro[4.4]nonane-8(S)-carboxylic acid, hydrobromide (prepared in paragraph E next above) in 100 ml of absolute methanol. Add 0.5 g of 2-oxo-4-phenylbutyric acid, ethyl ester and 10 ml of 3Å molecular sieve pellets and stir the mixture at room temperature for eighteen hours. Filter the reaction mixture and treat the filtrate with 0.30 g of sodium cyanoborohydride at room temperature for two hours. Concentrate the mixture under nitrogen and dilute the oil with 5% hydrochloric acid to pH 2 to 4 and stir at room temperature for one hour. Adjust the pH of the solution to pH 8 with 2.5N sodium hydroxide solution and absorb the solution in 150 ml of a XAD—2 resin. Elute the resin with 800 ml of water and then with 800 ml of methanol. Concentrate the methanol solution, place the residue on a column of silica gel (100 g, 60—200 mesh) and elute with chloroform:isopropanol:7% ammonium hydroxide 1:1:1 (organic layer) to obtain 7-[N-(1-carboethoxy-3-phenylpropyl)-(S)-alanyl]1,4-dithia-7-azaspiro[4.4]nonane-8(S)-carboxylic acid, a white solid, m.p. 56—60°C, $[\alpha]_D^{26}$ -25.5° (ethanol).

Example 9

7[N-(1-carboxy-3-phenylpropyl)-(S)-alanyl]-1,4-dithia-7-azaspiro[4.4]nonan-8(S)-carboxylic acid

Hydrolize 0.18 g of 7-[N-(1-carboethoxy-3-phenylpropyl)-(S)-alanyl]-1,4-dithia-7-azaspiro[4.4]nonane-8-(S)-carboxylic acid (prepared as described in Example 8) in 600 ml of methanol with 10 ml of 2.5N sodium

hydroxide, concentrate the reaction mixture and absorb it on an XAD—2 resin column and elute with water and then with methanol. Concentrate the methanol eluant to give a residue and absorb this residue on a silica gel column (100 g, 60—200 mesh). Elute the column with chlooroform:methanol: 14% ammonium hydroxide 1:1:1 and concentrate the desired eluant fractions to obtain the title compound.

Example 10

7-[N-(1-carbomethoxy-3-methylthiopropyl)-(R,S)-alanyl]-1,4-dithia-7-azaspiro[4.4]nonane-8(S)-carboxylic acid

A. Couple 1,4-dithia-7-azaspiro[4.4]nonane-8(S)-carboxylic acid, methyl ester (prepared as described in Example 8) with pyruvic acid using dicyclohexylcarbodimide and triethylamine in dioxane to yield, after isolation and hydrolysis of the ester, 7-pyruvoyl-1,4-dithia-7-azaspiro[4.4]nonane-8(S)-carboxylic acid.

B. Condense 7-pyruvoyl-1,4-dithia-7-azaspiro[4.4]nonane-8(S)-carboxylic acid and (S)-methionine, methyl ester with sodium cyanoborohydride in methanol at pH 7 for three days at room temperature followed by chromatography on a XAD—2 resin column, using methanol as eluant, to obtain the title compound.

Example 11

7-[N-(1-carboxy-3-methylthiopropyl)-(R,S)-alanyl]-1,4-dithia-7-azaspiro[4.4]nonane-8(S)-carboxylic acid

Treat 7-[N-(1-carbomethoxy-3-methylthiopropyl)-(R,S)-alanyl]-1,4-dithia-7-azaspiro[4.4]nonane-8(S)-carboxylic acid (prepared as described in Example 10) with sodium hydroxide in methanol as described in Example 9) to yield the title compound.

Example 12

7-{N-[1-carbomethoxy-2-(3-indolyl)ethyl]-(R,S)-alanyl}-1,4-dithia-7-azaspiro[4.4]nonane-8(S)-carboxylic acid

Use 7-pyruvoyl-1,4-dithia-7-azaspiro[4.4]nonane-8(S)-carboxylic acid (prepared as described in Example 10) and condense with tryptophan methyl ester in the presence of sodium cyanoborohydride using the method described in Example 10) to obtain the title compound.

Example 13

7-{N-[1-carboxy-2-(3-indolyl)ethyl]-(R,S)-alanyl}-1,4-dithia-7-azaspiro[4.4]nonane-8(S)-carboxylic acid

Hydrolize 7-{-N-[1-carbomethoxy-2-(3-indolyl)ethyl]-(R,S)-alanyl}-1,4-dithia-7-azaspiro[4.4]nonane-8(S)-carboxylic acid (prepared as described in Example 12) with sodium hydroxide as described in Example 9) to yield the title compound.

Example 14

7-{N-[1-carbomethoxy-2-(1H-imidazol-4-yl)ethyl]-(R,S)alanyl}-1,4-dithia-7-azaspiro[4.4]nonane-8(S)-carboxylic acid

As described in Example 10, react 7-pyruvoyl-1,4-dithia-7-azaspiro[4.4]nonane-8(S)-carboxylic acid (prepared as described in Example 10) and (S)-histidine, methyl ester in the presence of sodium cyanoborohydride to obtain the title compound.

Example 15

7-{N-[1-carboxy-2-(1H-imidazol-4-yl)ethyl]-(R,S)-alanyl}-1,4-dithia-7-azaspiro[4.4]nonane-8(S)-carboxylic acid

Treat 7-{N-[1-carbomethoxy-2-(1*H*-imidazolyl-4-yl)-ethyl]-(R,S)-alanyl}-1,4-dithia-7-azaspiro[4.4]-nonane-8(S)-carboxylic acid (prepared as described in Example 14) with sodium hydroxide as described in Example 9) to yield the title compound.

Example 16

7-[N-(1-carboethoxy-3-phenylpropyl)glycyl]-1,4-dithia-7-azaspiro[4.4]nonane-8(S)-carboxylic acid

A. As described in Example 8, react 1-benzyloxycarbonyl-4-keto-(S)-proline, ethyl ester (prepared from the acid by esterification in ethanol) with 1,2-ethanedithiol to obtain 7-benzyloxycarbonyl-1,4-dithia-7-azaspiro[4.4]nonane-8(S)-carboxylic acid, ethyl ester, a yellow oil $[\alpha]_D^{26}$ - 21.0° (ethanol).

B. Convert 2.22 g of 7-benzyloxycarbonyl-1,4-dithia-7-azaspiro[4.4]nonane-8(S)-carboxylic acid, ethyl ester (prepared as described in paragraph A) to 1,4-dithia-7-azaspiro[4.4]nonane-8(S)-carboxylic acid, ethyl ester as described in Example 8 and couple this compound with 1.5 g of N-benzyloxycarbonylglycine, N-hydroxysuccinimide ester as described in Example 8) to yield 7-(N-benzyloxycarbonylglycyl)-1,4-dithia-7-azaspiro[4.4]nonane-8(S)-carboxylic acid, ethyl ester, a yellow oil $[\alpha]_D^{26}$ - 21.0°.

C. Hydrolize 1.43 g of 7-(N-benzyloxycarbonylglycyl)-1,4-dithia-7-azaspiro[4.4]nonane-8(S)-carboxylic acid, ethyl ester (prepared as described in paragraph B next above) with sodium hydroxide as described in Example 8 to obtain 7-(N-benzyloxycarbonylglycyl)-1,4-dithia-7-azaspiro[4.4]nonane-8(S)-carboxylic acid. a colorless oil, $[\alpha]_D^{26}$ - 7.9°.

D. Treat 0.95 g of the acid obtained in the process described in paragraph C next above with 20% hydrobromic acid in glacial acetic acid as described in Example 8) obtain 7-glycyl-1,4-dithia-7-azaspiro-[4.4]-nonane-8(S)-carboxylic acid, hydrobromide $[\alpha]_D^{26}$ - 18.7°

E. As described in Example 8, couple 0.76 g of 7-glycyl-1,4-dithia-7-azaspiro[4.4]nonane-8(S)-carboxylic acid, hydrobromide (prepared as described in paragraph D next above) with 0.50 g of 2-oxo-4-phenylbutyric acid, ethyl ester to obtain 7-[N-(1-carboethoxy-3-phenylpropyl)-glycyl]-1,4-dithia-7-azaspiro-[4.4]nonane-8(S)-carboxylic acid.

Example 17

7-[N-(1-carboxy-3-phenylpropyl)glycyl]-1,4-dithia-7-azaspiro[4.4]nonane-8(S)-carboxylic acid

As described in Example 9, hydrolize 7-[N-(1-carboethoxy-3-phenylpropyl)glycyl]-1,4-dithia-7-azaspiro-[4.4]-nonane-8(S)-carboxylic acid (prepared as described in Example 31) with sodium hydroxide to give the title compound.

Example 18

7-(N-(1-carboethoxy-3-phenylpropyl)-(S)-alanyl]-1,4-dithia-7-azaspiro[4.5]decane-8(S)-carboxylic acid

A. Dissolve 1-benzyloxycarbonyl-5-hydroxy-(S)-pipecolic acid (prepared from 5-hydroxy-(S)-pipecolic acid in 2N sodium hydroxide solution treated with benzylchlorformate in diethyl ether) in acetone and treat with Jones reagent to obtain 1-benzyloxycarbonyl-5-keto-(S)-pipecolic acid. Then esterify in methanol to give the respective methyl ester.

B. Substitute 1-benzyloxycarbonyl-5-keto-(S)-pipecolic acid, methyl ester as the keto-ester in Example 8 and follow the procedure described to obtain the title compound.

Example 19

7-[N-(1-carboxy-3-phenylpropyl(-(S)-alanyl]-1,4-dithia-7-azaspiro[4.5]decane-8(S)-carboxylic acid

Hydrolize 7[N-(1-carboethoxy-3-phenylpropyl)-(S)-alanyl]-1,4-dithia-7-azaspiro[4.5]decane-8(S)-carboxylic acid (prepared as described in Example 18) with sodium hydroxide and isolate the title compound using the procedure described in Example 9.

Example 20

1-[N-(1-carboethoxy-3-phenylpropyl)-(S)-alanyl]-1-azaspiro[4.4]nonane-2(S)-carboxylic acid.

A. Condense nitrocyclopentane (prepared from bromocyclopentane and sodium nitrite) and acrolein in tetrahydrofuran in the presence of sodium hydride to obtain 3-(1-nitrocyclopentyl)propionaldehyde. Treat this aldehyde with p-toluene-sulfonic acid in methanol and isolate 3-(1-nitrocyclopentyl)propionaldehyde

dimethyl acetal. Hydrogenate this compound with Raney nickel. Isolate 3-(1-aminocyclopentyl)-propionaldehyde dimethylacetal and dissolve in aqueous acetone in the presence of p-toluenesulfonic acid and heat under reflux, followed by addition of toluene and azeotrope the mixture to give 1-azaspiro-[4.4]-$\Delta^1$-nonane.

B. Substitute 1-azaspiro[4.4]-$\Delta^1$-nonane (from paragraph A) for cis-hexahydro-$\Delta^1$-isoindole in Example 3A to obtain 1-azaspiro[4.4]nonane-2-carboxylic acid.

C. Use 1-azaspiro[4.4]nonane-2-carboxylic acid, ethyl ester prepared by esterification of the acid (obtained as described in paragraph B) in methanol in place of octahydroindole-2-carboxylic acid, ethyl ester in Preparation 1B through 1E to obtain the title compound.

Example 21

2-[N-(1-carboethoxy-3-phenylpropyl)-(S)-alanyl]-2-azaspiro[4.4]nonane-3(S)-carboxylic acid

A. Reduce 2-(1-cyanocyclopentylacetaldehyde diethyl acetal (preparable from 3-cyanopropionaldehyde diethyl acetal and 1,4-dibromobutane in tetrahydrofuran in the presence of sodium hydride) with lithium aluminum hydride to yield 2-(1-aminomethylcyclopenryl)acetaldehye, diethyl acetal. Dissolve this compound in aqueous acetone in the presence of p-toluenesulfonic acid and heat under reflux, followed by addition of toluene and azeotrope the mixture to give $\Delta^2$-2-azaspiro[4.4]nonane.

B. Substitute $\Delta^2$-2-azaspiro[4.4]nonane (prepared as described in paragraph A for *cis*-hexahydro-$\Delta^1$-isoindole in Example 3A to yield 2-azaspiro[4.4]nonane-3-carboxylic acid.

C. As described in Preparation 1B through 1E, substitute 2-azaspiro[4.4]nonane-3-carboxylic acid, ethyl ester (preparable by esterfication of the acid from paragraph B in ethanol) for octahydroindole-2-carboxylic acid, ethyl ester to obtain the title compound.

The following compounds exemplify the compounds of formula I, which can be prepared according to the described processes:

1-[N-(1-carbomethoxy-3-phenylpropyl)-(S)-alanyl]-decahydrocyclohepta[b]pyrrole-2(S)-carboxylic acid;
7-[N-(1-carboethoxy-3-phenylpropyl)-(S)-alanyl]-1-thia-7-azaspiro[4.4]nonane-8(S)-carboxylic acid;
1-[N-(1-carbomethoxy-3-phenylpropyl)-(S)-alanyl]-octahydrocyclopenta[b]pyrrole-2(S)-carboxylic acid;
1-[N-(1-carboethoxy-3-phenylpropyl)-(S)-alanyl]-octahydrocyclopenta[c]pyrrole-2(S)-carboxylic acid;
1-[N-(1-carboxy-3-phenylpropyl)-(S)-alanyl]-octahydrocyclopenta[c]pyrrole-2(S)-carboxylic acid;
1-[N-(1-carbomethoxy-3-phenylpropyl)-(S)-alanyl)-decahydroquinoline-2(S)-carboxylic acid;
1-[N-(1-carboxy-3-phenylpropyl)glycyl]-decahydroquinoline-2(S)-carboxylic acid;
1-[N-(1-carboethoxy-3-p-chlorophenylpropyl)-(S)-alanyl]-decahydroquinoline-2(S)-carboxylic acid;
1-[N-(1-carboxy-2-phenylethyl)-(S)-alanyl]-octahydroisoindole-1(S)-carboxylic acid;
5-[N-(1-carboxy-3-phenylpropyl)-(S)-alanyl]-2,2-dimethyloctahydro-1,3-dioxolo[4,5-c]pyrrole-4(S)-carboxylic acid;
6-[N-(1-carboxy-3-phenylpropyl)-(S)-alanyl]-octahydro-1,4-dioxino[2,3-c]pyrrole-5(S)-carboxylic acid;
5-[N-(1-carboxy-3-phenylpropyl)-(S)-alanyl]-octahydro-1,3-dithiolo[4,5-c]pyrrole-4(S)-carboxylic acid;
6-[N-(1-carboxy-3-phenylpropyl)-(S)-alanyl]-octahydro-1,4-dithiino[2,3-c]pyrrole-5(S)-carboxylic acid;
5-[N-(1-carboxy-3-phenylpropyl)-(S)-alanyl]-2,2-dimethyloctahydro-1,3-dioxolo[4,5-c]pyridine-6(S)-carboxylic acid;
6-[N-(1-carboxy-3-phenylpropyl)-(S)-alanyl]-decahydro-1,4-dioxino[4,5-c]pyridine-7(S)-carboxylic acid;
5-[N-(1-carboxy-3-phenylpropyl)-(S)-alanyl]-octahydro-1,3-dithiolo[4,5-c]pyridine-6(S)-carboxylic acid;
6-[N-(1-carboxy-3-phenylpropyl)-(S)-alanyl]-decahydro-1,4-dithiino[2,3-c]pyridine-7(S)-carboxylic acid;
5-[N-(1-carboxy-3-phenylpropyl)-(S)-alanyl]-octahydro-1,3-dioxolo[4,5-c]pyridine-4(S)-carboxylic acid;
6-[N-(1-carboxy-3-phenylpropyl)-(S)-alanyl]-decahydro-1,4-dioxino[2,3-c]pyridine-5(S)-carboxylic acid;
5-[N-(1-carboxy-3-phenylpropyl)-(S)-alanyl]-octahydro-1,3-dithiolo[4,5-c]pyridine-4(S)-carboxylic acid;
6-[N-(1-carboxy-3-phenylpropyl)-(S)-alanyl]-decahydro-1,4-dithiino[2,3-c]pyridine-5(S)-carboxylic acid;
7-[N-(1-carboethoxy-3-p-chlorophenylpropyl)-(S)-alanyl]-1,4-dithia-7-azaspiro[4.4]nonane-8(S)-carboxylic acid;
7-[N-(1-carboxy-3-p-chlorophenylpropyl)-(S)-alanyl]-1,4-dithia-7-azaspiro[4.4]nonane-8(S)-carboxylic acid;
7-[N-(1-carboxy-2-phenoxyethyl)-(S)-alanyl]-1,4-dithia-7-azaspiro[4.4]nonane-8(S)-carboxylic acid; 7-[N-(1-carbomethoxy-3-methylthiopropyl)-(R,S)-alanyl]-1,4-dithia-7-azaspiro[4.4]nonane-8(S)-carboxylic acid;
7-[N-(1-carboxy-3-methylthiopropyl)-(R,S)-alanyl]-1,4.dithia-7-azaspiro[4.4]nonane-8(S)-carboxylic acid;
7-{N-[1-carboethoxy-2-(3-indolyl)ethyl]-(R,S)-alanyl}-1,4-dithia-7-azaspiro[4.4]nonane-8(S)-carboxylic acid;

7-{N-[1-carboxy-2-(3-indolyl)ethyl]-(R,S)-alanyl}-1,4-dithia-7-azaspiro[4.4]nonane-8(S)-carboxylic acid;
7-{N-[1-carboethoxy-2-(1H-imidazol-4-yl)ethyl]-(R,S)-alanyl}-1,4-dithia-7-azaspiro[4.4]nonane-8(S)-carboxylic acid;
7-[N-(1-carbomethoxy-3-phenylpropyl)glycyl]-1,4-dithia-7-azaspiro[4.4]nonane-8(S)-carboxylic acid;
7-[N-(1-carboethoxy-3-phenylpropyl)-(S)-alanyl]-1,4-dioxa-7-azaspiro[4.4]nonane-8(S)carboxylic acid;
2-[N-(1-carboethoxy-3-phenylpropyl)-(S)-alanyl]-6,10-dioxa-2-azaspiro[4.5]decane-3(S)carboxylic acid;
2-[N-(1-carboethoxy-3-phenylpropyl)-(S)-alanyl]-6,10-dithia-2-azaspiro[4.5]decane-3(S)-carboxylic acid;
7-[N-(1-carboethoxy-3-phenylpropyl)-(S)-alanyl]-1,4-dioxa-7-azaspiro[4.4]nonane-6(S)-carboxylic acid;
2-[N-(1-carboethoxy-3-phenylpropyl)-(S)-alanyl]-6,10-dioxa-2-azaspiro[4.5]decane-1(S)-carboxylic acid;
7-[N-(1-carboethoxy-3-phenylpropyl)-(S)-alanyl]-1,4-dithia-7-azaspiro[4.4]nonane-6(S)-carboxylic acid;
2-[N-(1-carboethoxy-3-phenylpropyl)-(S)-alanyl]-6,10-dithia-2-azaspiro[4.5]decane-1(S)-carboxylic acid;
7-[N-(1-carboethoxy-3-phenylpropyl)-(S)-alanyl]-1,4-dioxa-7-azaspiro[4.5]decane-8(S)-carboxylic acid;
7-[N-(1-carboethoxy-3-phenylpropyl)-(S)-alanyl]-1,4-dithia-7-azaspiro[4.5]decane-8(S)-carboxylic acid;
8-[N-(1-carboethoxy-3-phenylpropyl)-(S)-alanyl]-1,5-dithia-8-azaspiro[5.5]undecane-9(S)-carboxylic acid;
8-[N-(1-carboethoxy-3-phenylpropyl)-(S)-alanyl]-1,4-dioxa-8-azaspiro[4.5]decane-7(S)-carboxylic acid;
9-[N-(1-carboethoxy-3-phenylpropyl)-(S)-alanyl]-1,5-dioxa-9-azaspiro[5.5]undecane-8(S)-carboxylic acid;
8-[N-(1-carboethoxy-3-phenylpropyl)-(S)-alanyl]-1,4-dithia-8-azaspiro[4.5]decane-7(S)-carboxylic acid;
9-[N-(1-carboethoxy-3-phenylpropyl)-(S)-alanyl]-1,5-dithia-9-azaspiro[5.5]undecane-8(S)-carboxylic acid:
1-[N-(1-carboethoxy-3-phenylpropyl)-(S)-alanyl]-1-azaspiro[4.5]decane-2(S)-carboxylic acid;
2-[N-(1-carboethoxy-3-phenylpropyl)-(S)-alanyl]-2-azaspiro[4.5]decane-3(S)-carboxylic acid;
2-[N-(1-carboethoxy-3-phenylpropyl)-(S)-alanyl]-2-azaspiro[4.5]decane-7(S)-carboxylic acid;
2-[N-(1-carboethoxy-3-phenylpropyl)-(S)-alanyl]-7-azaspiro[4.5]decane-8(S)-carboxylic acid;
2-[N-(1-carboethoxy-3-phenylpropyl)-(S)-alanyl]-2-azaspiro[5.5]undecane-3(S)-carboxylic acid;
8-[N-(1-carboethoxy-3-phenylpropyl)-(S)-alanyl]-8-azaspiro[4.5]decane-7(S)-carboxylic acid;
3-[N-(1-carboethoxy-3-phenylpropyl)-(S)-alanyl]-3-azaspiro[5.5]undecane-2(S)-carboxylic acid;
7-[N-(1-carboethoxy-3-phenylpropyl)-(S)-alanyl]-1-oxa-7-azaspiro[4.4]nonane-8(S)-carboxylic acid;
7-[N-(1-carboethoxy-3-phenylpropyl)-(S)-alanyl]-1-oxa-7-azaspiro[4.4]nonane-6(S)-carboxylic acid;
7-[N-(1-carboethoxy-3-phenylpropyl)-(S)-alanyl]-1-oxa-7-azaspiro[4.5]decane-8(S)-carboxylic acid;
8-[N-(1-carboethoxy-3-phenylpropyl)-(S)-alanyl]-1-oxa-8-azaspiro[5.5]undecane-9(S)-carboxylic acid;
8-[N-(1-carboethoxy-3-phenylpropyl)-(S)-alanyl]-1-oxa-8-azaspiro[4.5]decane-7(S)-carboxylic acid;
9-[N-(1-carboethoxy-3-phenylpropyl)-(S)-alanyl]-1-oxa-9-azaspiro[5.5]undecane-8(S)-carboxylic acid;
7-[N-(1-carboethoxy-2-benzyloxyethyl)-(S)-alanyl]-1,4-dithia-7-azaspiro[4.4]nonane-8(S)-carboxylic acid;
7-[N-(1-carboethoxy-2-benzylthioethyl)-(S)-alanyl]-1,4-dithia-7-azaspiro[4.4]nonane-8(S)-carboxylic acid;
1-[N-(1-carboethoxy-2-benzyloxyethyl)-(S)-alanyl]decahydrocyclohepta[b]pyrrole-2(S)-carboxylic acid;
1-[N-(1-carboethoxy-2-benzylthioethyl)-(S)-alanyl]-decahydrocyclohepta[b]pyrrole-2(S)-carboxylic acid;
7-[N-(1(S)-carboethoxy-3-phenylpropyl-(S)-alanyl]-1,4-dithia-7-azaspiro[4.4]nonane-8(S)-carboxylic acid;
$[\alpha]_D^{26}$ = -39.0° 7-[N-(3-phenyl-1-ethoxycarbonylpropyl)glycyl]-1,4-dithia-7-azaspiro[4.4]nonane-(S)-carboxylic acid;
$[\alpha]_D^{26}$ = -2.4° (ETOH) 1-[N-(1-carboethoxy-3-phenylpropyl)-(S)-alanyl]-perhydrocyclopenta[b]pyrrole-2-( )-carboxylic acid;
$[\alpha]_D^{26}$ = -5.8° (ETOH) 1-[N-1-carboethoxy-3-phenylpropyl)-(S)-alanyl]-perhydrocyclopenta[b]pyrrole-2-( )-carboxylic acid.

The following examples describe in detail composition that are illustrative of the present invention. It will be apparent to those skilled in the art that many modifications, both of materials and methods, may be practiced without departing from the purpose and intent of this disclosure.

| Formulation 1 | | |
|---|---|---|
| Capsule | Amount mg | |
| Active ingredient | 250.0 | 125.0 |
| Lactose | 173.0 | 86.5 |
| Corn Starch | 75.0 | 37.5 |
| Magnesium Stearate | 2.0 | 1.0 |
| | 500.0 | 250.0 |

Blend the active ingredient, lactose and corn starch until uniform; then blend the magnesium stearate into the resulting powder. Encapsulate the mixture into suitably sized tow-piece hard gelatin capsules.

| Formulation 2 | | |
|---|---|---|
| Tablet | Amount (mg) | |
| Active ingredient | 250.0 | 125.0 |
| Lactose | 161.0 | 80.5 |
| Corn Starch | 12.0 | 6.0 |
| Water (per thousand tablets) | 120 ml (evaporates) | 60 ml (evaporates) |
| Corn Starch | 75.0 | 37.5 |
| Magnesium Stearate | 2.0 | 1.0 |
| | 500.0 | 250.0 |

Blend the active ingredient with the lactose until uniform. Blend the smaller quantity of corn starch with the water and add the resulting coorn starch paste, then mix until a uniform wet mass is formed. Add the remaining corn starch to the remaining wet mass and mix until uniform granules are obtained. Screen the granules through a suitable milling machine, using a 3/4 inch stainless steel screen. Dry the milled granules in a suitable drying oven until the desired moisture content is obtained. Mill the dried granules through a suitable milling machine using a 16 mesh stainless steel screen. Blend in the magnesium stearate and compress the resulting mixture into tablets opf desired shape, thickness, hardness and disintegration.

| Formulation 3 | |
|---|---|
| Injectable Solution | mg/ml |
| Active ingredient | 5.00 |
| Methyl-*p*-hydroxybenzoate | 0.80 |
| Propyl-*p*-hydroxybenzoate | 0.10 |
| Disodium Edetate | 0.10 |
| Citric Acid Monohydrate | 0.08 |
| Dextrose | 40.0 |
| Water for injection qs. ad. | 1.0 ml |

Dissolve the *p*-hydroxybenzoates in a portion of water for injection at 60—70 °C and cool the solution to 25—35 °C. Charge and dissolve all other excipients and the active ingredient. Bring the solution to a final volume, filter it through a sterilizing membrane and fill into sterile containers.

**Claims**

**Claims for the following Contracting States : BE, CH, DE, FR, GB, IT, LI, LU, NL, SE**

**1.** A compound of the formula

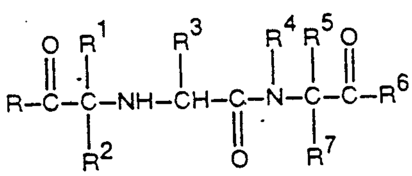

I

or a pharmaceutically acceptable salt thereof, wherein R and $R^6$ are the same or different and are hydroxy, lower alkoxy, lower alkenyloxy, dilower alklylamino lower alkoxy, acylamino lower alkoxy, acyloxy lower alkoxy, aryloxy, aryllower alkoxy, amino, lower alkylamino, dilower alkylamino, hydroxyamino, aryllower alkylamino, or substituted aryloxy or substituted aryllower alkoxy wherein the substituent is methyl, halo or methoxy; $R^1$ is hydrogen, alkyl of from 1 to 10 carbon atoms, substituted lower alkyl wherein the substituent is hydroxy, lower alkoxy, aryloxy, substituted aryloxy, heteroaryloxy, substituted heteroaryloxy, amino, lower alkylamino, diloweralkylamino, acylamino, arylamino, substituted arylamino, guanidino, imidazolyl, indolyl, lower alkylthio, arylthio, substituted arylthio, carboxy, carbamoyl, lower alkoxy carbonyl, aryl, substituted aryl, aralkyloxy, substituted aralkyloxy, aralkylthio or

substituted aralkylthio, wherein the aryl or heteroaryl portion of said substituted aryloxy, heteroaryloxy, arylamino, arylthio, aryl, aralkyloxy, aralkylthio group is substituted with a group selected from halo, lower alkyl, hydroxy, lower alkoxy, amino, aminomethyl, carboxyl, cyano, or sulfamoyl; $R^2$ and $R^7$ are the same or different and are hydrogen or lower ally; $R^3$ is hydrogen, lower ally, phenyl lower alkyl, aminomethylphenyl lower alkyl, hydroxyphenyl lower alkyl, hydroxy lower ally. acylamino lower alkyl, amino lower alkyl, dimethylamino lower alkyl, guanidino lower alkyl, imidazolyl lower alkyl, indolyl lower alkyl, or lower alkyl thio lower alkyl; $R^4$ and $R^5$ taken together form a group represented by Q or U, wherein;

Q is

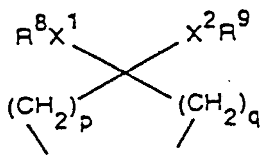

wherein $X^1$ and $X^2$ independent of each other are O, S or $CH_2$, $R^8$ and $R^9$ taken together form a bridge W, wherein W is an alkylene or substituted alkylene bridge having 2 or 3 carbon atoms, said substituted alkylene bridge having one or two substituents selected from lower alkyl, aryl lower alkyl groups, p is 0, 1 or 2, q is 0. 1 or 2, with the proviso that the sum of p and q must be 1, 2 or 3, with the proviso that if p is 0 then $X^1$ and $X^2$ must be methylene;

U is

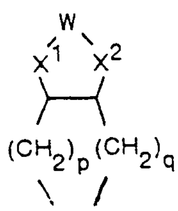

, wherein W is a methylene bridge or a substituted methylene bridge when at least one of $X^1$ and $X^2$ is methylene, or W is an alkylene or substituted alkylene bridge having 2 or 3 carbon atoms, said substituted methylene bridge or said substituted alkylene bridge having one or two substituents selected from lower alkyl, aryl and aryl lower alkyl groups, (except that W may also be a methylene bridge when $X^1$ and $X^2$ are oxygen or sulphur), $X^1$ and $X^2$ are as defined above, p is 0, 1 or 2, q is 0, 1 or 2, with the proviso that the sum of p and q is 1 or 2, and with the proviso that if p is 0. $X^1$ must be $CH_2$, excluding compounds wherein U is

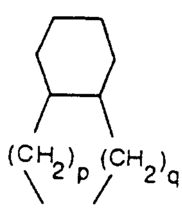

wherein p is zero or 1 and q is 1.

**2.** A compound according to claim 1, wherein $R^4$ and $R^5$ taken together form the group Q, wherein $X^1$ amd $X^2$ are methylene, $R^8$ and $R^9$ taken together form the bridge W, W preferablky being an ethylene bridge, and wherein p and q are each 1 or wherein p is 0 and q is 2; or wherein $X^1$ and $X^2$ are S, and $R^8$ and $R^9$ taken together form an ethylene bridge and p and a preferably are each 1 or p is 1 and q is 2.

**3.** A compound according to claim 1, wherein $R^4$ and $R^5$ taken together form the group U, wherein $X^1$ and $X^2$ are methylene and W is methylene and p preferably is zero and a preferably is 1 or 2; or wherein $X^1$ and $X^2$ are methylene and W is ethylene and wherein preferably p is 0 and q is 2 or p is 1 and q is 0; or wherein $X^1$ and $X^2$ are methylene and W is trimethylene and wherein preferably p is 0 and q is 1; or wherein $X^1$ and $X^2$ are O, W is methylene and p and q are as defined in claim 1.

**4.** A compound according to any one of claims 1 to 3, wherein $R^1$ is substituted lower alkyl; wherein the substituent is unsubstituted or lower-alkyl-substituted aryl, aralkyloxy or aralkylthio, $R^1$ preferably being substituted lower alkyl, wherein the substituent is aralkyloxy or aralkylthio, preferably benzyloxy or benzylthio.

**5.** A compound according to any one of claims 1 to 4, wherein R and $R^6$ are the same or different and are hydroxy, lower alkoxy or aryllower alkoxy; $R^2$ and $R^7$ are hydrogen; and $R^3$ is hydrogen, lower alkyl or phenyl lower alkyl, preferably R being hydroxy, methoxy or ethoxy; $R^6$ being hydroxy, ethoxy or benzyloxy; $R^2$ and $R^7$ being hydrogen; and $R^3$ being hydrogen, methyl or benzyl.

**6.** A compound according to claim 2, wherein $X^1$ and $X^2$ are S, $R^8$ and $R^9$ taken together form an ethylene bridge and p and q are each 1, $R^1$ is phenethyl, R and $R^6$ are the same or different and are hydroxy or ethoxy, $R^2$ and $R^7$ are hydrogen and $R^3$ is hydrogen or methyl.

**7.** A compound according to claim 6, which is

7-[N-(1-carboethoxy-3-phenylpropyl)-(S)-alanyl-1,4-dithia-7-azaspiro[4.4]nonane-8(S)-carboxylic acid,

7-[N-(1-carboxy-3-phenylpropyl)-(S)-alanyl]-1,4-dithia-7-azaspiro[4.4]nonane-8(S)-carboxylic acid,

7-[N-(1-carboethoxy-3-phenylpropyl)glycyl]-1,4-dithia-7-azaspiro[4.4]nonane-8(S)carboxylic acid or

7-[N-(1-carboxy-3-phenylpropyl)glycyl]-1,4-dithia-7-azaspiro[4.4]nonane-8(S)-carboxylic acid.

**8.** A compound according to any one of claims 1 to 7, wherein the aminoacid part structures have the S-configuration.

**9.** A compound according to claim 7 or 8, which is 7-[N-(1(S)-carboethoxy-3-phenylpropyl)-(S)-alanyl]-1,4-dithia-7-azaspiro[4.4]nonane-8(S)-carboxylic acid.

**10.** Process for the preparation of a compound of formula I as defined in any one of claim 1 to 9, characterized in that the compound is prepared by an appropriate process selected from the following processes (whereby in the following formulae R, $R^1$, $R^2$, $R^3$, $R^4$, $R^5$, $R^6$ and $R^7$ are as defined for formula I, including suitable protection):

a) for the preparation of compounds of formula I wherein $R^2$ is hydrogen) condensation of a ketocompound (XIII with a dipeptide (XIV) under reduction

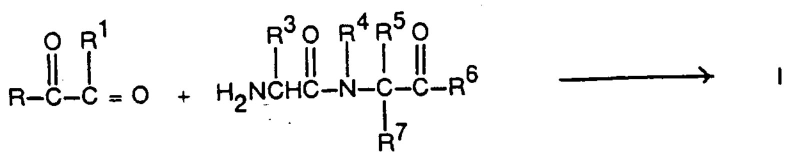

XIII XIV

b) alkylation of a dipeptide (XIV) by means of a compound (XXII)

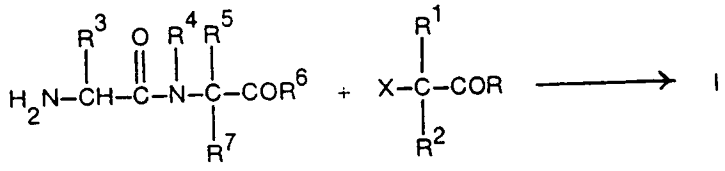

XIV XXII

wherein X is chloro, bromo, iodo, alkanesulfonyloxy or arenesulfonyloxy;

c) condensation of an amino compound (XVIII) with a ketocompound (XIX) under reduction

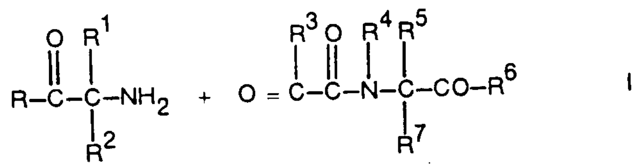

XVIII XIX

d) alkylation of an amino compound (XVIII) by means of a compound (XXIII)

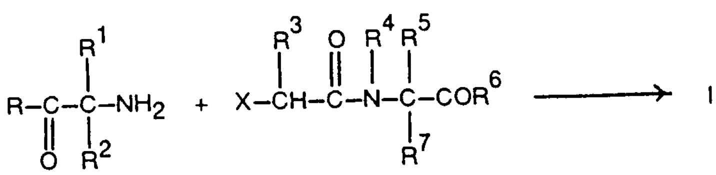

XVIII XXIII

wherein X is chloro, bromo, iodo, alkanesulfonyloxy or arenesulfonyloxy;

e) condensation of an aminoacid (XXI) with an aminoacid (XVII)

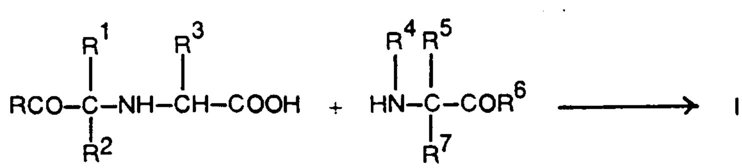

XXI XVII

followed by removal of the protecting groups if necessary to yield the desired product, and if desired, converting a so obtained compound of formula I into another compound of formula I, and, if desired, preparing a salt thereof and, if desired, isolating the preferred isomer.

**11.** A pharmaceutical composition useful in the treatment of hypertension, comprising a compound of the general formula I as defined in any one of claims 1 to 9 or a pharmaceutically acceptable salt thereof, preferably in admixture with a suitable pharmaceutically acceptable carrier or excipient.

## Claims for the following Contracting State : AT

1. Process for the preparation of a compound of the general formula

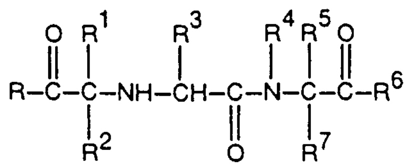

I

or a pharmaceutically acceptable salt thereof, wherein R and $R^6$ are the same or different and are hydroxy, lower alkoxy, lower alkenyloxy, dilower alkylamino lower alkoxy, acylamino lower alkoxy, acyloxy lower alkoxy, aryloxy, aryllower alkoxy, amino, lower alkylamino, dilower alkylamino, hydroxyamino; aryllower alkylamino, or substituted aryloxy or substituted aryllower alkoxy wherein the substituent is methyl, halo or methoxy; $R^1$ is hydrogen, alkyl of from 1 to 10 carbon atoms, substituted lower alkyl wherein the substituent is hydroxy, lower alkoxy, aryloxy, substituted aryloxy, heteroaryloxy, substituted heteroaryloxy, amino, lower alkylamino, diloweralkylamino, acylamino, arylamino, substituted arylamino, guanidino, imidazolyl, indolyl, lower alkylthio, arylthio, substituted arylthio, carboxy, carbamoyl, lower alkoxy carbonyl, aryl, substituted aryl, aralkyloxy, substituted aralkyloxy, aralkylthio or substituted aralkylthio, wherein the aryl or heteroaryl portion of said substituted aryloxy, heteroaryloxy, arylamino, arylthio, aryl, aralkyloxy, aralkylthio group is substituted with a group selected from halo, lower alkyl, hydroxy, lower alkoxy, amino, aminomethyl, carboxyl, cyano, or sulfamoyl; $R^2$ and $R^7$ are the same or different and are hydrogen or lower alkyl; $R^3$ is hydrogen, lower alkyl, phenyl lower alkyl, aminomethylphenyl lower alkyl, hydroxyphenyl lower alkyl, hydroxy lower alkyl. acylamino lower alkyl, amino lower alkyl, dimethylamino lower alkyl, guanidino lower alkyl, imidazolyl lower alkyl, indolyl lower alkyl, or lower alkyl thio lower alkyl; $R^4$ and $R^5$ taken together form a group represented by Q or U, wherein;

Q is

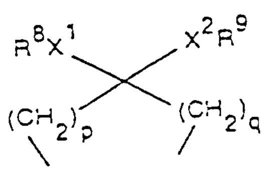

wherein $X^1$ and $X^2$ independent of each other are O, S or $CH_2$, $R^8$ and $R^9$ taken together form a bridge W, wherein W is an alkylene or substituted alkylene bridge having 2 or 3 carbon atoms, said substituted alkylene bridge having one or two substituents selected from lower alkyl, aryl and aryl lower alkyl groups, p is 0, 1 or 2, q is 0. 1 or 2, with the proviso that the sum of p and q must be 1, 2 or 3, with the proviso that if p is 0 then $X^1$ and $X^2$ must be methylene;

U is

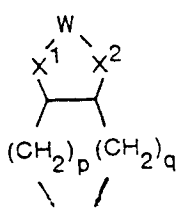

, wherein W is a methylene bridge or a substituted methylene bridge when at least one of $X^1$ and $X^2$ is methylene, or W is an alkylene or substituted alkylene bridge having 2 or 3 carbon atoms, said substituted methylene bridge or said substituted alkylene bridge having one or two substituents

selected from lower alkyl, aryl and aryl lower alkyl groups, (except that W may also be a methylene bridge when $X^1$ and $X^2$ are oxygen or sulphur), $X^1$ and $X^2$ are as defined above, p is 0, 1 or 2, q is 0, 1 or 2, with the proviso that the sum of p and q is 1 or 2, and with the proviso that if p is 0. $X^1$ must be $CH_2$, excluding compounds wherein U is

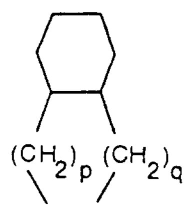

wherein p is zero or 1 and q is 1;
characterised in that the compound is prepared by an appropriate process selected from the following processes (whereby in the following formulae R, $R^1$, $R^2$, $R^3$, $R^4$, $R^5$, $R^6$ and $R^7$ are as defined for formula I, including suitable protection):

a) for the preparation of compounds of formula I wherein $R^2$ is hydrogen) condensation of a ketocompound (XIII with a dipeptide (XIV) under reduction

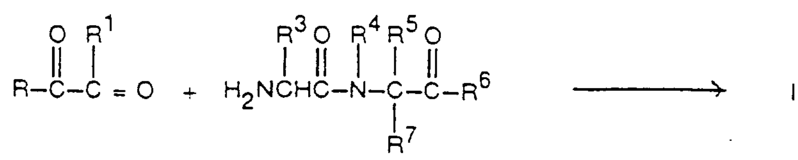

XIII XIV

b) alkylation of a dipeptide (XIV) by means of a compound (XXII)

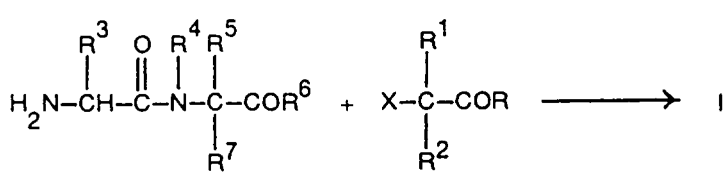

XIV XXII

wherein X is chloro, bromo, iodo, alkanesulfonyloxy or arenesulfonyloxy;

c) condensation of an amino compound (VIII) with a ketocompound (XIX) under reduction

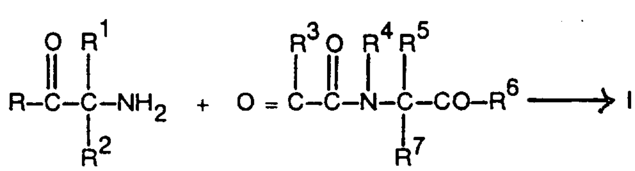

XVIII XIX

d) alkylation of an amino compound (XVIII) by means of a compound (XXIII)

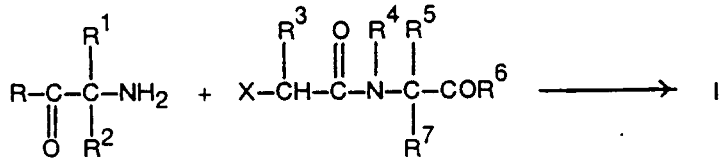

XVIII XXIII

wherein X is chloro, bromo, iodo, alkanesulfonyloxy or arenesulfonyloxy;

e) condensation of an aminoacid (XXI) with an aminoacid (XVII)

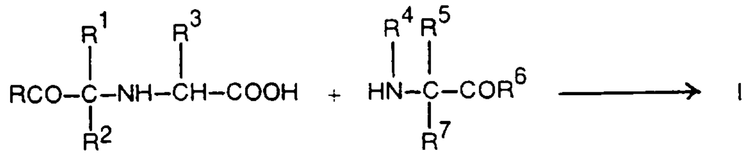

XXI XVII

followed by removal of the protecting groups if necessary to yield the desired product, and if desired, converting a so obtained compound of formula I into another compound of formula I, and, if desired, preparing a salt thereof and, if desired, isolating the preferred isomer.

**2.** Process according to claim 1, characterized in that a compound of formula I is prepared wherein $R^4$ and $R^5$ taken together form the group Q, wherein $X^1$ amd $X^2$ are methylene, $R^8$ and $R^9$ taken together form the bridge W, W preferably being an ethylene bridge, and wherein p and q are each 1 or wherein p is 0 and q is 2; or wherein $X^1$ and $X^2$ are S, and $R^8$ and $R^9$ taken together form an ethylene bridge and p and q preferably are each 1 or p is 1 and q is 2.

**3.** Process according to claim 1, characterized in that a compound of formula I is prepared wherein $R^4$ and $R^5$ taken together form the group U, wherein $X^1$ and $X^2$ are methylene and W is methylene and p preferably is zero and q preferably is 1 or 2; or wherein $X^1$ and $X^2$ are methylene and W is ethylene and wherein preferably p is 0 and q is 2 or p is 1 and q is 0; or wherein $X^1$ and $X^2$ are methylene and W is trimethylene and wherein preferably p is 0 and q is 1; or wherein $X^1$ and $X^2$ are O, W is methylene and p and q are as defined in claim 1.

**4.** Process according to any one of claims 1 to 3, characterized in that a compound of formula I is prepared wherein $R^1$ is substituted lower alkyl; wherein the substituent is unsubstituted or lower-alkyl-substituted aryl, aralkyloxy or aralkylthio, $R^1$ preferably being substituted lower alkyl, wherein the substituent is aralkyloxy or aralkylthio, preferably benzyloxy or benzylthio.

**5.** Process according to any one of claims 1 to 4, characterized in that a compound of formula I is prepared wherein R and $R^6$ are the same or different and are hydroxy, lower alkoxy or aryllower alkoxy; $R^2$ and $R^7$ are hydrogen; and $R^3$ is hydrogen, lower alkyl or phenyl lower alkyl, preferably R being hydroxy, methoxy or ethoxy; $R^6$ being hydroxy, ethoxy or benzyloxy; $R^2$ and $R^7$ being hydrogen; and $R^3$ being hydrogen, methyl or benzyl.

**6.** Process according to claim 2, characterized in that a compound of formula I is prepared wherein $X^1$ and $X^2$ are S, $R^8$ and $R^9$ taken together form an ethylene bridge and p and q are each 1, $R^1$ is phenethyl, R and $R^6$ are the same or different and are hydroxy or ethoxy, $R^2$ and $R^7$ are hydrogen and $R^3$ is hydrogen or methyl.

7. Process ccording to claim 9, characteized in that a compound is prepared which is 7-[N-(1-carboethoxy-3-phenylpropyl)-(S)-alanyl-1,4-dithia-7-azaspiro[4.4]nonane-8(S)-carboxylic acid, 7-[N-(1-carboxy-3-phenylpropyl)-(S)-alanyl]-1,4-dithia-7-azaspiro[4.4]nonane-8(S)-carboxylic acid, 7-[N-(1-carboethoxy-3-phenylpropyl)glycyl]-1,4-dithia-7-azaspiro[4.4]nonane-8(S)carboxylic acid or 7-[N-(1-carboxy-3-phenylpropyl)glycyl]-1,4-dithia-7-azaspiro[4.4]nonane-8(S)-carboxylic acid.

8. Process according to any one of claims 1 to 7, characterized in that a compound is prepared wherein the aminoacid part structures have the S-configuration.

9. Process according to claim 7 or 8, characterized in that a compound is prepared which is 7-[N-(1(S)-carboethoxy-3-phenylpropyl)-(S)-alanyl]-1,4-dithia-7-azaspiro[4.4]nonane-8(S)-carboxylic acid.

10. Process for preparing pharmaceutical compositions useful in the treatment of hypertension, characterized in that a compound of the general Formula I as defined in any one of claims 1 to 9 or a pharmaceutically acceptable salt thereof, is brought into a form suitable for pharmaceutical administration.

**Patentansprüche**
**Patentansprüche für folgende Vertragsstaaten : BE, CH, DE, FR, GB, IT, LI, LU, NL, SE**

1. Verbindung der Formel

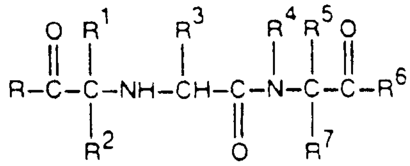

I

oder ein pharmazeutisch annehmbares Salz derselben, worin

| | |
|---|---|
| R und $R^6$ | gleich oder verschieden sind und Hydroxy, Niederalkoxy, Niederalkenyloxy, Diniederalkylamino-niederalkoxy, Acylamino-niederalkoxy, Acyloxy-niederalkoxy, Aryloxy, Arylniederalkoxy, Amino, Niederalkylamino, Diniederalkylamino, Hydroxyamino, Arylniederalkylamino oder substituiertes Aryloxy oder substituiertes Arylniederalkoxy sind, worin der Substituent Methyl, Halogen oder Methoxy ist; |
| $R^1$ | Wasserstoff, Alkyl mit 1 bis 10 Kohlenstoff-Atomen, substituiertes Niederalkyl, worin der Substituent Hydroxy, Niederalkoxy, Aryloxy, substituiertes Aryloxy, Heteroaryloxy, substituiertes Heteroaryloxy, Amino, Niederalkylamino, Diniederalkylamino, Acylamino, Arylamino, substituiertes Arylamino, Guanidino, Imidazolyl, Indolyl, Niederalkylthio, Arylthio, substituiertes Arylthio, Carboxy, Carbamoyl, Niederalkoxycarbonyl, Aryl, substituiertes Aryl, Aralkyloxy, substituiertes Aralkyloxy, Aralkylthio oder substituiertes Aralkylthio ist, worin der Aryl- oder Heteroaryl-Teil dieser substituierten Aryloxy-, Heteroaryloxy-, Arylamino-, Arylthio-, Aryl-, Aralkyloxy-, Aralkylthio-Gruppe mit einer aus Halogen, Niederalkyl, Hydroxy, Niederalkoxy, Amino, Aminomethyl, Carboxyl, Cyano oder Sulfamoyl ausgewählten Gruppe substituiert ist; |
| $R^2$ und $R^7$ | gleich oder verschieden sind und Wasserstoff oder Niederalkyl sind; |
| $R^3$ | Wasserstoff,Niederalkyl,Phenylniederalkyl,Aminomethylphenyl-niederalkyl, Hydroxyphenyl-niederalkyl, Hydroxyniederalkyl, Acylaminoniederalkyl, Aminoniederalkyl, Dimethylaminoniederalkyl, Guanidinoniederalkyl;Imidazolylniederalkyl, Indolylniederalkyl oder Niederalkylthioniederalkyl ist; |
| $R^4$ und $R^5$ | zusammengenommen eine durch Q oder U bezeichnete Gruppe bilden, worin |
| Q | |

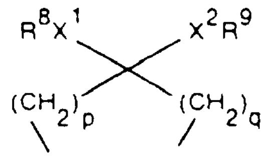

ist, worin $X^1$ und $X^2$ unabhängig voneinander O, S oder $CH_2$ sind,

$R^8$ und $R^9$ zusammengenommen eine Brücke W bilden, worin W eine Alkylen- oder substituierte Alkylen-Brücke mit 2 oder 3 Kohlenstoff-Atomen ist, wobei die substituierte Alkylen-Brücke einen oder zwei Substituenten ausgewählt aus Niederalkyl-, Aryl- und Arylniederalkyl-Gruppen aufweist,

p 0, 1 oder 2 ist,

q 0, 1 oder 2 ist, mit der Maßgabe, daß die Summe aus p und q 1, 2 oder 3 sein muß, mit der Maßgabe, daß, wenn

p 0 ist, dann $X^1$ und $X^2$ Methylen sein müssen;

U

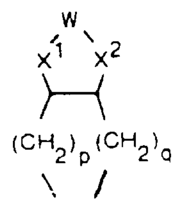

ist, worin

W eine Methylen-Brücke oder eine substituierte Methylen-Brücke ist, wenn wenigstens einer der Substituenten $X^1$ oder $X^2$ Methylen ist, oder W eine Alkylen- oder substituierte Alkylen-Brücke mit 2 oder 3 Kohlenstoff-Atomen ist, wobei die substituierte Methylen-Brücke oder die substituierte Alkylen-Brücke einen oder zwei Substituenten ausgewählt aus Niederalkyl-, Aryl- und Arylniederalkyl-Gruppen aufweist (mit der Ausnahme, daß W auch eine Methylen-Brücke sein kann, wenn $X^1$ und $X^2$ Sauerstoff oder Schwefel sind),

$X^1$ und $X^2$ die im Vorstehenden angegebenen Bedeutungen haben,

p 0, 1 oder 2 ist,

q 0, 1 oder 2 ist, mit der Maßgabe, daß die Summe aus p und q 1 oder 2 ist, und mit der Maßgabe, daß, wenn p 0 ist, $X^1$ $CH_2$ sein muß, ausschließlich solcher Verbindungen, in denen

U

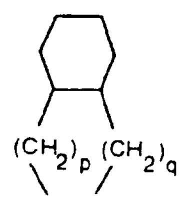

ist, worin p Null oder 1 ist und q 1 ist.

**2.** Verbindung nach Anspruch 1, dadurch gekennzeichnet, daß $R^4$ und $R^5$ zusammengenommen die Gruppe Q bilden, worin

$X^1$ und $X^2$ Methylen sind,

| | |
|---|---|
| $R^8$ und $R^9$ | zusammengenommen die Brücke W bilden, wobei W vorzugsweise eine Ethylen-Brücke ist, und |
| p und q | jeweils 1 sind oder worin |
| p | 0 ist und q 2 ist, oder worin |
| $X^1$ und $X^2$ | S sind und |
| $R^8$ und $R^9$ | zusammengenommen eine Ethylen-Brücke bilden und |
| p und q | vorzugsweise jeweils 1 sind oder |
| p | 1 ist und q 2 ist. |

**3.** Verbindung nach Anspruch 1, dadurch gekennzeichnet, daß $R^4$ und $R^5$ zusammengenommen die Gruppe U bilden, worin

| | |
|---|---|
| $X^1$ und $X^2$ | Methylen sind und |
| W | Methylen ist und |
| p | vorzugsweise Null ist und |
| q | vorzugsweise 1 oder 2 ist oder worin |
| $X^1$ und $X^2$ | Methylen sind und |
| W | Ethylen ist und worin vorzugsweise |
| p | 0 ist und |
| q | 2 ist oder |
| p | 1 ist und |
| q | 0 ist oder worin |
| $X^1$ und $X^2$ | Methylen sind und |
| W | Trimethylen ist und worin vorzugsweise |
| p | 0 ist und |
| q | 1 ist oder worin |
| $X^1$ und $X^2$ | O sind, |
| W | Methylen ist und |
| p und q | die in Anspruch 1 angegebenen Bedeutungen haben. |

**4.** Verbindung nach irgendeinem der Ansprüche 1 bis 3, dadurch gekennzeichnet, daß $R^1$ substituiertes Niederalkyl ist, worin der Substituent unsubstituiertes oder niederalkyl-substituiertes Aryl, Aralkyloxy oder Aralkylthio ist, wobei $R^1$ vorzugsweise substituiertes Niederalkyl ist, worin der Substituent Aralkyloxy oder Aralkylthio ist, vorzugsweise Benzyloxy oder Benzylthio.

**5.** Verbindung nach irgendeinem der Ansprüche 1 bis 4, dadurch gekennzeichnet, daß R und $R^6$ gleich oder verschieden sind und Hydroxy, Niederalkoxy oder Arylniederalkoxy sind, $R^2$ und $R^7$ Wasserstoff sind und $R^3$ Wasserstoff, Niederalkyl oder Phenylniederalkyl ist, wobei vorzugsweise R Hydroxy, Methoxy oder Ethoxy ist, $R^6$ Hydroxy, Ethoxy oder Benzyloxy ist, $R^2$ und $R^7$ Wasserstoff sind und $R^3$ Wasserstoff, Methyl oder Benzyl ist.

**6.** Verbindung nach Anspruch 2, dadurch gekennzeichnet, daß

| | |
|---|---|
| $X^1$ und $X^2$ | S sind, |
| $R^8$ und $R^9$ | zusammengenommen eine Ethylen-Brücke bilden und |
| p und q | jeweils 1 sind, |
| $R^1$ | Phenethyl ist, |
| R und $R^6$ | gleich oder verschieden sind und Hydroxy oder Ethoxy sind, |
| $R^2$ und $R^7$ | Wasserstoff sind und |
| $R^3$ | Wasserstoff oder Methyl ist. |

**7.** Verbindung nach Anspruch 6, die
7-[N-(1-Carboethoxy-3-phenylpropyl)-(S)-alanyl]-1,4-dithia-7-azaspiro[4.4]nonan-8(S)-carbonsäure,
7-[N-(1-Carboxy-3-phenylpropyl)-(S)-alanyl]-1,4-dithia-7-azaspiro[4.4]nonan-8(S)-carbonsäure,
7-[N-(1-Carboethoxy-3-phenylpropyl)-glycyl]1,4-dithia-7-azaspiro[4.4]nonan-8(S)-carbonsäure oder
7-[N-(1-Carboxy-3-phenylpropyl)-glycyl]-1,4-dithia-7-azaspiro[4.4]nonan-8(S)-carbonsäure ist.

**8.** Verbindung nach irgendeinem der Ansprüche 1 bis 7, dadurch gekennzeichnet, daß die Aminosäure-Teilstrukturen die S-Konfiguration besitzen.

9. Verbindung nach Ansprüchen 7 oder 8, die 7-[N-(1(S)-Carboethoxy-3-phenylpropyl)-(S)-alanyl]-1,4-dithia-7-azaspiro[4.4]nonan-8(S)-carbonsäure ist.

10. Verfahren zur Herstellung einer Verbindung der Formel I nach irgendeinem der Ansprüche 1 bis 9, dadurch gekennzeichnet, daß die Verbindung mit Hilfe eines passenden Verfahrens hergestellt wird, das aus den nachstehenden Verfahren ausgewählt ist (wobei in den folgenden Formeln R, $R^1$, $R^2$, $R^3$, $R^4$, $R^5$, $R^6$ und $R^7$ die für die Formel I angegebenen Bedeutungen, einschließlich geeigneten Schutzes, haben):

a) zur Herstellung von Verbindungen der Formel I, in denen $R^2$ Wasserstoff ist, Kondensation einer Keto-Verbindung (XIII) mit einem Dipeptid (XIV) unter Reduktion

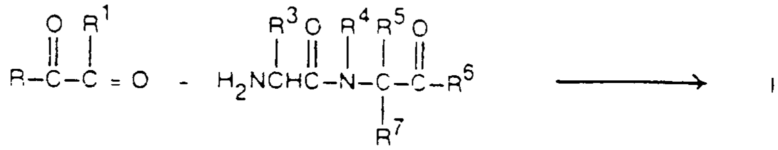

XIII XIV ;

b) Alkylierung eines Dipeptids (XIV) mit Hilfe einer Verbindung (XXII)

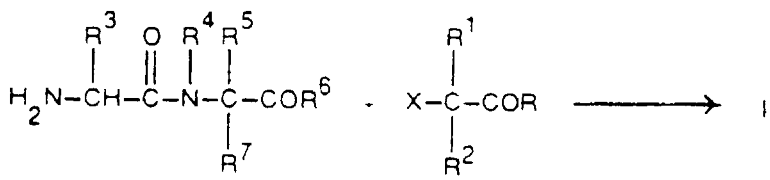

XIV XXII ;

worin X Chlor, Brom, Iod, Alkansulfonyloxy oder Arensulfonyloxy ist;

c) Kondensation einer Amino-Verbindung (XVIII) mit einer Keto-Verbindung (XIX) unter Reduktion

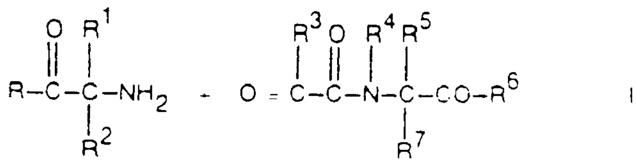

XVIII XIX ;

d) Alkylierung einer Amino-Verbindung (XVIII) mit Hilfe einer Verbindung (XXIII),

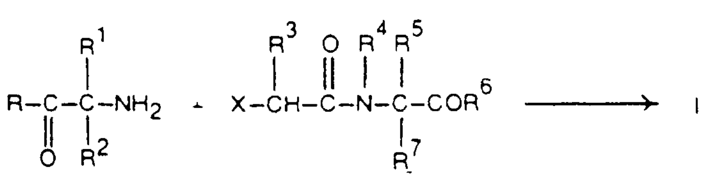

XVIII XXIII ;

worin X Chlor, Brom, Iod, Alkansulfonyloxy oder Arensulfonyloxy ist;

e) Kondensation einer Aminosäure (XXI) mit einer Aminosäure (XVII)

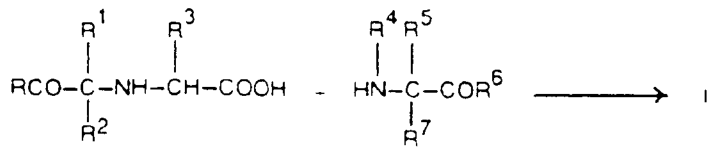

XXI XVII

und nachfolgende Entfernung der Schutzgruppen, falls erforderlich, wodurch das gewünschte Produkt erhalten wird, und gewünschtenfalls Überführen der so erhaltenen Verbindung der Formel I in eine andere Verbindung der Formel I und gewünschtenfalls Herstellen eines Salzes derselben sowie gewünschtenfalls Isolierung des bevorzugten Isomers.

**11.** Pharmazeutische Zusammensetzung, die für die Behandlung von Hypertonie geeignet ist, enthaltend eine Verbindung der allgemeinen Formel I nach irgendeinem der Ansprüche 1 bis 9 oder ein pharmazeutisch annehmbares Salz derselben, vorzugsweise im Gemisch mit einem geeigneten pharmazeutisch annehmbaren Träger oder Exzipienten.

**Patentansprüche für folgenden Vertragsstaat : AT**

**1.** Verfahren zur Herstellung einer Verbindung der allgemeinen Formel

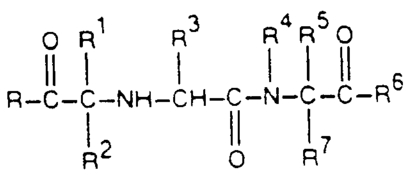

I

oder eines pharmazeutisch annehmbaren Salzs derselben, worin

R und $R^6$ gleich oder verschieden sind und Hydroxy, Niederalkoxy, Niederalkenyloxy, Diniederalkylamino-niederalkoxy, Acylamino-niederalkoxy, Acyloxy-niederalkoxy, Aryloxy, Arylniederalkoxy, Amino, Niederalkylamino, Diniederalkylamino, Hydroxyamino, Arylniederalkylamino oder substituiertes Aryloxy oder substituiertes Arylniederalkoxy sind, worin der Substituent Methyl, Halogen oder Methoxy ist;

$R^1$ Wasserstoff, Alkyl mit 1 bis 10 Kohlenstoff-Atomen, substituiertes Niederalkyl, worin der Substituent Hydroxy, Niederalkoxy, Aryloxy, substituiertes Aryloxy, Heteroaryloxy, substituiertes Heteroaryloxy, Amino, Niederalkylamino, Diniederalkylamino, Acylamino, Arylamino, substituiertes Arylamino, Guanidino, Imidazolyl, Indolyl, Niederalkylthio, Arylthio, substituiertes Arylthio, Carboxy, Carbamoyl, Niederalkoxycarbonyl, Aryl, substituiertes Aryl, Aralkyloxy, substituiertes Aralkyloxy, Aralkylthio oder substituiertes Aralkylthio ist, worin der Aryl- oder Heteroaryl-Teil dieser substituierten Aryloxy-, Heteroaryloxy-, Arylamino-, Arylthio-, Aryl-, Aralkyloxy-, Aralkylthio-Gruppe mit einer aus Halogen, Niederalkyl, Hydroxy, Niederalkoxy, Amino, Aminomethyl, Carboxyl, Cyano oder Sulfamoyl ausgewählten Gruppe substituiert ist;

$R^2$ und $R^7$ gleich oder verschieden sind und Wasserstoff oder Niederalkyl sind;

$R^3$ Wasserstoff,Niederalkyl,Phenylniederalkyl,Aminomethylphenyl-niederalkyl, Hydroxyphenyl-niederalkyl, Hydroxyniederalkyl, Acylaminoniederalkyl, Aminoniederalkyl, Dimethylaminoniederalkyl, Guanidinoniederalkyl, Imidazolylniederalkyl, Indolylniederalkyl oder Niederalkylthioniederalkyl ist;

$R^4$ und $R^5$ zusammengenommen eine durch Q oder U bezeichnete Gruppe bilden, worin

Q

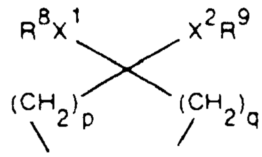

ist, worin $X^1$ und $X^2$ unabhängig voneinander O, S oder $CH_2$ sind,

R⁸ und R⁹ zusammengenommen eine Brücke W bilden, worin W eine Alkylen- oder substituierte Alkylen-Brücke mit 2 oder 3 Kohlenstoff-Atomen ist, wobei die substituierte Alkylen-Brücke einen oder zwei Substituenten ausgewählt aus Niederalkyl-, Aryl- und Arylniederalkyl-Gruppen aufweist,

p 0, 1 oder 2 ist,

q 0, 1 oder 2 ist, mit der Maßgabe, daß die Summe aus p und q 1, 2 oder 3 sein muß, mit der Maßgabe, daß, wenn

p 0 ist, dann $X^1$ und $X^2$ Methylen sein müssen;

U

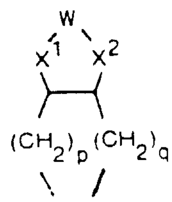

ist, worin

W eine Methylen-Brücke oder eine substituierte Methylen-Brücke ist, wenn wenigstens einer der Substituenten $X^1$ oder $X^2$ Methylen ist, oder W eine Alkylen- oder substituierte Alkylen-Brücke mit 2 oder 3 Kohlenstoff-Atomen ist, wobei die substituierte Methylen-Brücke oder die substituierte Alkylen-Brücke einen oder zwei Substituenten ausgewählt aus Niederalkyl-, Aryl- und Arylniederalkyl-Gruppen aufweist (mit der Ausnahme, daß W auch eine Methylen-Brücke sein kann, wenn $X^1$ und $X^2$ Sauerstoff oder Schwefel sind),

$X^1$ und $X^2$ die im Vorstehenden angegebenen Bedeutungen haben,

p 0, 1 oder 2 ist,

q 0, 1 oder 2 ist, mit der Maßgabe, daß die Summe aus p und q 1 oder 2 ist, und mit der Maßgabe, daß, wenn p 0 ist, $X^1$ $CH_2$ sein muß, ausschließlich solcher Verbindungen, in denen

U

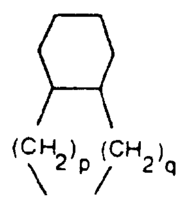

ist, worin p Null oder 1 ist und q 1 ist,

dadurch gekennzeichnet, daß die Verbindung mit Hilfe eines passenden Verfahrens hergestellt wird, das aus den nachstehenden Verfahren ausgewählt ist (wobei in den folgenden Formeln R, $R^1$, $R^2$, $R^3$, $R^4$, $R^5$, $R^6$ und $R^7$ die für die Formel I angegebenen Bedeutungen, einschließlich geeigneten Schutzes, haben):

a) zur Herstellung von Verbindungen der Formel I, in denen $R^2$ Wasserstoff ist, Kondensation einer Keto-Verbindung (XIII) mit einem Dipeptid (XIV) unter Reduktion

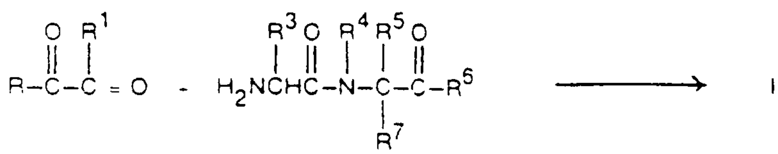

XIII XIV ;

b) Alkylierung eines Dipeptids (XIV) mit Hilfe einer Verbindung (XXII)

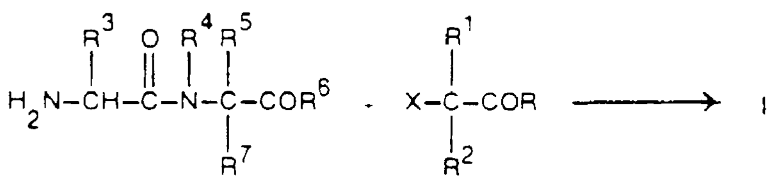

XIV XXII ;

worin X Chlor, Brom, Iod, Alkansulfonyloxy oder Arensulfonyloxy ist;
c) Kondensation einer Amino-Verbindung (XVIII) mit einer Keto-Verbindung (XIX) unter Reduktion

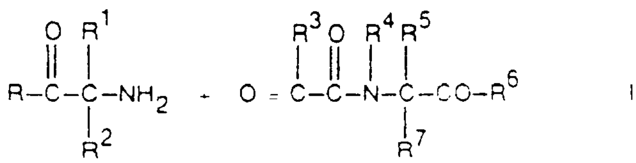

XVIII XIX ;

d) Alkylierung einer Amino-Verbindung (XVIII) mit Hilfe einer Verbindung (XXIII),

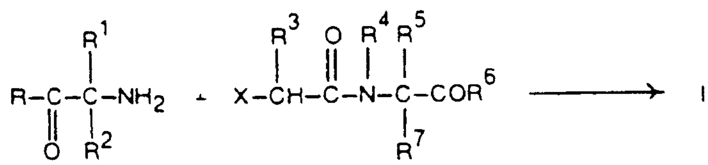

XVIII XXIII ;

worin X Chlor, Brom, Iod, Alkansulfonyloxy oder Arensulfonyloxy ist;

e) Kondensation einer Aminosäure (XXI) mit einer Aminosäure (XVII)

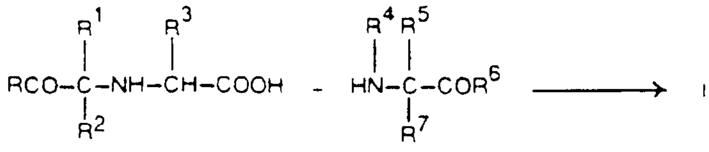

XXI XVII

und nachfolgende Entfernung der Schutzgruppen, falls erforderlich, wodurch das gewünschte Produkt erhalten wird, und gewünschtenfalls Überführen der so erhaltenen Verbindung der Formel I in eine andere Verbindung der Formel I und gewünschtenfalls Herstellen eines Salzes derselben sowie gewünschtenfalls Isolierung des bevorzugten Isomers.

**2.** Verfahren nach Anspruch 1, dadurch gekennzeichnet, daß eine Verbindung der Formel I hergestellt wird, worin $R^4$ und $R^5$ zusammengenommen die Gruppe Q bilden, worin

| | |
|---|---|
| $X^1$ und $X^2$ | Methylen sind, |
| $R^8$ und $R^9$ | zusammengenommen die Brücke W bilden, wobei W vorzugsweise eine Ethylen-Brücke ist, und |
| p und q | jeweils 1 sind oder worin |
| p | 0 ist und q 2 ist, oder worin |
| $X^1$ und $X^2$ | S sind und |
| $R^8$ und $R^9$ | zusammengenommen eine Ethylen-Brücke bilden und |
| p und q | vorzugsweise jeweils 1 sind oder |
| p | 1 ist und q 2 ist. |

**3.** Verfahren nach Anspruch 1, dadurch gekennzeichnet, daß eine Verbindung der Formel I hergestellt wird, worin $R^4$ und $R^5$ zusammengenommen die Gruppe U bilden, worin

| | |
|---|---|
| $X^1$ und $X^2$ | Methylen sind und |
| W | Methylen ist und |
| p | vorzugsweise Null ist und |
| q | vorzugsweise 1 oder 2 ist oder worin |
| $X^1$ und $X^2$ | Methylen sind und |
| W | Ethylen ist und worin vorzugsweise |
| p | 0 ist und |
| q | 2 ist oder |
| p | 1 ist und |
| q | 0 ist oder worin |
| $X^1$ und $X^2$ | Methylen sind und |
| W | Trimethylen ist und worin vorzugsweise |
| p | 0 ist und |
| q | 1 ist oder worin |
| $X^1$ und $X^2$ | O sind, |
| W | Methylen ist und |
| p und q | die in Anspruch 1 angegebenen Bedeutungen haben. |

**4.** Verfahren nach irgendeinem der Ansprüche 1 bis 3, dadurch gekennzeichnet, daß eine Verbindung der Formel I hergestellt wird, worin $R^1$ substituiertes Niederalkyl ist, worin der Substituent unsubstituiertes oder niederalkyl-substituiertes Aryl, Aralkyloxy oder Aralkylthio ist, wobei $R^1$ vorzugsweise substituiertes Niederalkyl ist, worin der Substituent Aralkyloxy oder Aralkylthio ist, vorzugsweise Benzyloxy oder Benzylthio.

**5.** Verfahren nach irgendeinem der Ansprüche 1 bis 4, dadurch gekennzeichnet, daß eine Verbindung der Formel I hergestellt wird, worin R und $R^6$ gleich oder verschieden sind und Hydroxy, Niederalkoxy oder Arylniederalkoxy sind, $R^2$ und $R^7$ Wasserstoff sind und $R^3$ Wasserstoff, Niederalkyl oder Phenylniederalkyl ist, wobei vorzugsweise R Hydroxy, Methoxy oder Ethoxy ist, $R^6$ Hydroxy, Ethoxy oder

Benzyloxy ist, $R^2$ und $R^7$ Wasserstoff sind und $R^3$ Wasserstoff, Methyl oder Benzyl ist.

**6.** Verfahren nach Anspruch 2, dadurch gekennzeichnet, daß eine Verbindung der Formel I hergestellt wird, worin

| | |
|---|---|
| $X^1$ und $X^2$ | S sind, |
| $R^8$ und $R^9$ | zusammengenommen eine Ethyen-Brücke bilden und |
| p und q | jeweils 1 sind, |
| R1 | Phenethyl ist, |
| R und $R^6$ | gleich oder verschieden sind und Hydroxy oder Ethoxy sind, |
| $R^2$ und $R^7$ | Wasserstoff sind und |
| $R^3$ | Wasserstoff oder Methyl ist. |

**7.** Verfahren nach Anspruch 6, dadurch gekennzeichnet, daß eine Verbindung hergestellt wird, die
7-[N-(1-Carboethoxy-3-phenylpropyl)-(S)-alanyl]-1,4-dithia-7-azaspiro[4.4]nonan-8(S)-carbonsäure,
7-[N-(1-Carboxy-3-phenylpropyl)-(S)-alanyl]-1,4-dithia-7-azaspiro[4.4]nonan-8(S)-carbonsäure,
7-[N-(1-Carboethoxy-3-phenylpropyl)-glycyl]-1,4-dithia-7-azaspiro[4.4]nonan-8(S)-carbonsäure oder
7-[N-(1-Carboxy-3-phenylpropyl)-glycyl]-1,4-dithia-7-azaspiro[4.4]nonan-8(S)-carbonsäure ist.

**8.** Verfahren nach irgendeinem der Ansprüche 1 bis 7, dadurch gekennzeichnet, daß eine Verbindung hergestellt wird, worin die Aminosäure-Teilstrukturen die S-Konfiguration besitzen.

**9.** Verfahren nach Anspruch 7 oder 8, dadurch gekennzeichnet, daß eine Verbindung hergestelltwird,die7-[N-(1(S)-Carboethoxy-3-phenylpropyl)-(S)-alanyl]-1,4-dithia-7-azaspiro[4.4]nonan-8(S)-carbonsäure ist.

**10.** Verfahren zur Herstellung pharmazeutischer Zusammensetzungen, die für die Behandlung von Hypertonie geeignet sind, dadurch gekennzeichnet, daß eine Verbindung der allgemeinen Formel I nach irgendeinem der Ansprüche 1 bis 9 oder ein pharmazeutisch annehmbares Salz derselben in eine für die pharmazeutische Verabreichung geeignete Form gebracht wird.

## Revendications

**Revendications pour les Etats contractants suivants : BE, CH, DE, FR, GB, IT, LI, LU, NL, SE**

**1.** Composé de formule

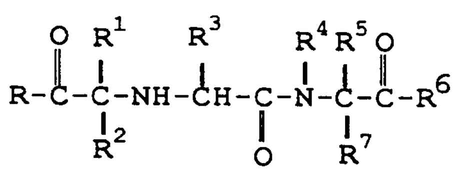

I

ou son sel acceptable en pharmacie, où R et $R^6$ sont identiques ou différents et sont hydroxy, alcoxy inférieur, alkényloxy inférieur, di-alcoylamino inférieur-alcoxy inférieur, acylamino-alcoxy inférieur, acyloxy-alcoxy inférieur, aryloxy, aryl-alcoxy inférieur, amino, alcoylamino inférieur, dialcoylamino inférieur, hydroxyamino, aryl-alcoylamino inférieur ou aryloxy substitué ou aryl-alcoxy inférieur substitué où le substituant est méthyle, halo ou méthoxy; $R^1$ est de l'hydrogène, un alcoyle de 1 à 10 atomes de carbone, un alcoyle inférieur substitué où le substituant est hydroxy, alcoxy inférieur, aryloxy, aryloxy substitué, hétéroaryloxy, hétéroaryloxy substitué, amino, alcoylamino inférieur, dialcoylamino inférieur, acylamino, arylamino, arylamino substitué, guanidino, imidazolyle, indoyle, alkylthio inférieur, arylthio, arylthio substitué, carboxy, carbamoyle, alcoxy inférieur-carbonyle, aryle, aryle substitué, aralcoyloxy, aralcoyloxy substitué, aralkylthio ou aralkylthio substitué, la partie aryle ou hétéroaryie dudit aryloxy, hétéroaryloxy, arylamino, arylthio, aryle, aralkyloxy ou aralkylthio substitué étant substituée par un groupe choisi parmi halo, alcoyle inférieur, hydroxy, alcoxy inférieur, amino, aminométhyle, carboxyle, cyano ou sulfamoyle; $R^2$ et $R^7$ sont identiques ou différents et sont de l'hydrogène ou un alcoyle inférieur; $R^3$ est hydrogène, alcoyle inférieur, phényl-alcoyle inférieur, aminométhylphényl-alcoyle inférieur, hydroxyphényl-alcoyle inférieur, hydroxy-alcoyle inférieur, acylamino-alcoyle inférieur, amino-alcoyle inférieur, diméthylamino-alcoyle inférieur, guanidino-alcoyle inférieur, imidazolyle-alcoyle inférieur, indolyl-alcoyle inférieur ou alcoyl inférieur-thio-alcoyle inférieur; $R^4$ et $R^5$ pris ensemble forment

un groupe représenté par Q ou U, où

Q est :

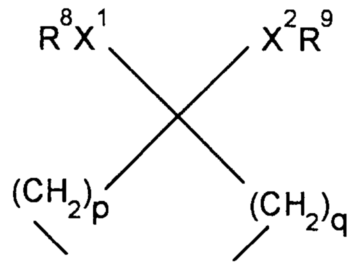

où $X^1$ et $X^2$ indépendamment l'un de l'autre sont O, S ou $CH_2$, $R^8$ et $R^9$ pris ensemble forment une liaison W, où W est un alcoylène ou un pont alcoylène substitué ayant 2 ou 3 atomes de carbone, ce pont alcoylène substitué ayant un ou plusieurs substituants choisis dans les groupes alcoyle inférieur, aryle et aryl -alcoyle inférieur, p est 0,1 ou 2, q est 0,1 ou 2, à condition que la somme de p et q doit être de 1, 2 ou 3, à condition que si p est O, alors $X^1$ et $X^2$ doivent être du méthylène.

U est

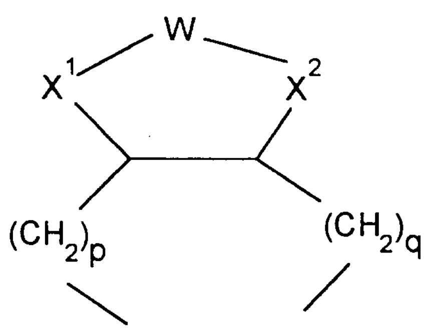

où W est un pont méthylène ou un pont méthylène substitué lorsqu'au moins l'un de $X^1$ et $X^2$ est méthylène, ou W est un pont alkylène ou alkylène substitué ayant 2 ou 3 atomes de carbone, ledit pour méthylène substitué ou ledit pour alkylène substitué ayant un ou deux substituants sélectionnés parmi les groupes alkyle inférieur, aryle et aryl alkyle inférieur (à l'exception que W peut également être un pont méthylène quand $X^1$ et $X^2$ sont de l'oxygène ou du soufre), $X^1$ et $X^2$ sont tels que définis ci-dessus, p est 0, 1 ou 2, q est 0, 1 ou 2, à condition que la somme de p et q soit 1 ou 2 et à la condition que si p est 0, $X^1$ doit être $CH_2$; à l'exclusion des composés où U est

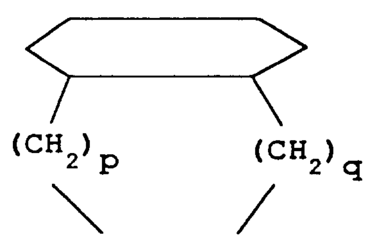

où p est zéro ou 1 et q est 1,

2. Composé selon la revendication 1, où $R^4$ et $R^5$ pris ensemble forment le groupe Q, où $X^1$ et $X^2$ sont méthylène, $R^8$ et $R^9$ pris ensemble forment le pont W, W étant de préférence un pont éthylène, et où p et q sont chacun de 1 ou bien où p est 0 et q est 2; ou bien où $X^1$ et $X^2$ sont S, et $R^8$ et $R^9$ pris ensemble forment un pont éthylène et p et q sont de préférence chacun 1 ou p est 1 et q est 2.

**3.** Composé selon la revendication 1, où $R^4$ et $R^5$ pris ensemble forment le groupe U, où $X^1$ et $X^2$ sont méthylène et W est méthylène et p est de préférence 1 ou 2; ou bien où $X^1$ et $X^2$ sont méthylène et W est éthylène et où de préférence p est 0 et q est 2 ou p est 1 et q est 0; ou bien $X^1$ et $X^2$ sont méthylène et W est triméthylène et où de préférence p est 0 et q est 1; ou bien $X^1$ et $X^2$ sont 0, W est méthylène et p est q sont tels que définis à la revendication 1.

**4.** Composé selon l'une quelconque des revendications 1 à 3, où $R^1$ est alcoyle inférieur substitué; où le substituant est aryle non substitué ou aryle substitué en alcoyle inférieur, aralcoyloxy ou aralkylthio, $R^1$ étant de préférence alcoyle inférieur substitué, où le substituant est aralcoyloxy ou aralkylthio, de préférence benzyloxy ou benzylthio.

**5.** Composé selon l'une quelconque des revendications 1 à 4, où R et $R^6$ sont identiques ou différents et sont hydroxy, alcoxy inférieur ou aryl-alcoxy inférieur; $R^2$ et $R^7$ sont hydrogène; et $R^3$ est hydrogène, alcoyle inférieur ou phényl-alcoyle inférieur, de préférence R étant hydroxy, méthoxy ou éthoxy; $R^6$ étant hydroxy, alcoxy ou benzyloxy; $R^2$ et $R^7$ étant hydrogène; et $R^3$ étant hydrogène, méthyle ou benzyle.

**6.** Composé selon la revendication 2, où $X^1$ et $X^2$ sont S, $R^8$ et $R^9$ pris ensemble forment un pont éthylène et p et q sont chacun 1, $R^1$ est phénéthyle, R et $R^6$ sont identiques ou différents et sont hydroxy ou éthoxy, $R^2$ et $R^7$ sont hydrogène et $R^3$ est hydrogène ou méthyle.

**7.** Composé selon la revendication 6 qui est l'acide 7-[N-(1-carboéthoxy-3-phénylpropyl)-(S)-alanyl-1,4-dithia-7-azaspiro [4,4] nonane-8(S)-carboxylique, l'acide 7-[N-(1-carboxy-3-phénylpropyl)-(S)-alanyl]-1,4-dithia-7-azaspiro [4,4] nonane-8(S)-carboxylique, l'acide 7-[N-(1-carboéthoxy-3-phénylpropyl)glycyl]-1,4-dithia-7-azaspiro [4,4] nonane-8(S)-carboxylique, l'acide 7-[N-(1-carboxy-3-phénylpropyl)glycyl]-1,4-dithia-7-azaspiro [4,4] nonane-8(S)-carboxylique.

**8.** Composé selon l'une quelconque des revendications 1 à 7, où les structures de la partie d'aminoacide ont la configuration S.

**9.** Composé selon la revendication 7 ou 8, qui est l'acide 7-[N-(1(S)-carboéthoxy-3-phénylpropyl)-(S)-alanyl] -1,4-dithia-7-azaspiro [4,4] nonane-8(S)-carboxylique.

**10.** Procédé de préparation d'un composé de formule I tel que défini selon la revendication 1 à 9, caractérisé en ce que le composé est préparé par un processus approprié choisi dans ceux qui suivent (dans les formules ci-après R, $R^1$, $R^2$, $R^3$, $R^4$, $R^5$, $R^6$ et $R^7$ sont tels que définis pour la formule I avec la protection appropriée) :

a) pour la préparation des composés de formule I où $R^2$ est de l'hydrogène, condensation d'un composé céto (XIII) avec un dipeptide (XIV) sous réduction

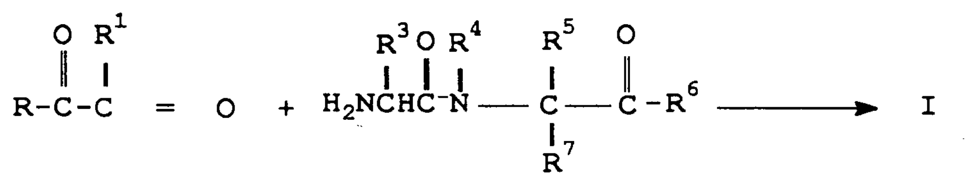

XIII XIV

b) alcoylation d'un dipeptide (XIV) au moyen d'un composé (XXII)

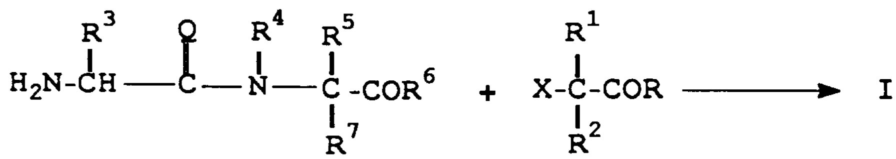

XIV XXII

où X est chloro, bromo, iodo, alcanesulfonyloxy ou arènesulfonyloxy;

c) condensation d'un composé amino (XVIII) avec un composé céto (XIX) sous réduction

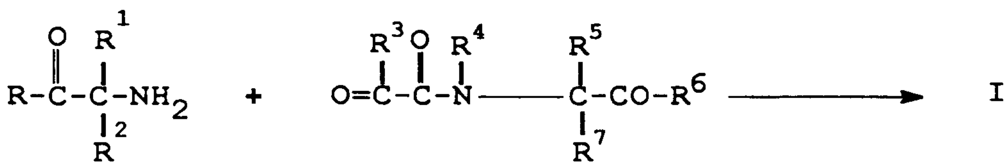

XVIII XIX

d) alcoylation d'un composé amino (XVIII) au moyen d'un composé (XXIII)

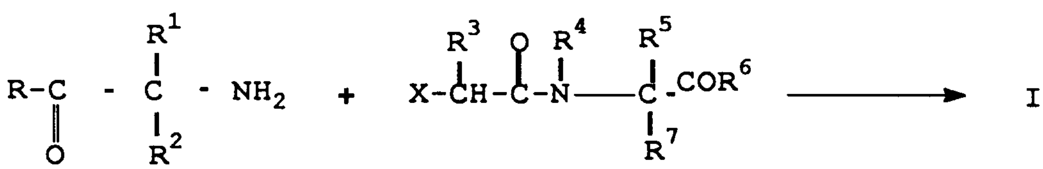

XVIII XXIII

où X est chloro, bromo, iodo, alcanesulfonyloxy ou arènesulfonyloxy;

e) condensation d'un aminoacide (XXI) avec un aminoacide (XVII)

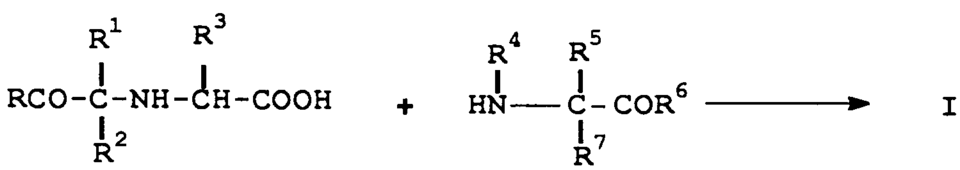

XXI XVII

suivie de l'enlèvement des groupes protecteurs si nécessaire pour donner le produit souhaité, et si on le souhaite, conversion d'un composé ainsi obtenu de formule I en un autre composé de formule I et si on le souhaite, préparation d'un sel et si on le souhaite isolement de l'isomère préféré.

**11.** Composition pharmaceutique utile pour le traitement de l'hypertension comprenant un composé de formule générale I tel que défini selon l'une des revendications 1 à 9, ou son sel acceptable en pharmacie, de préférence en mélange avec un véhicule ou excipient acceptable en pharmacie.

**Revendications pour l'Etat contractant suivant : AT**

**1.** Composé de formule

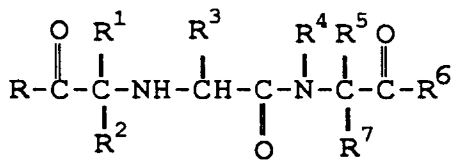

I

ou son sel acceptable en pharmacie, où R et $R^6$ sont identiques ou différents et sont hydroxy, alcoxy inférieur, alkényloxy inférieur, di-alcoylamino inférieur-alcoxy inférieur, acylamino-alcoxy inférieur, acyloxy-alcoxy inférieur, aryloxy, aryl-alcoxy inférieur, amino, alcoylamino inférieur, dialcoylamino inférieur, hydroxyamino, aryl-alcoylamino inférieur ou aryloxy substitué ou aryl-alcoxy inférieur substitué où le substituant est méthyle, halo ou méthoxy; $R^1$ est de l'hydrogène, un alcoyle de 1 à 10 atomes de carbone, un alcoyle inférieur substitué où le substituant est hydroxy, alcoxy inférieur, aryloxy, aryloxy substitué, hétéroaryloxy, hétéroaryloxy substitué, amino, alcoylamino inférieur, dialcoylamino inférieur, acylamino, arylamino, arylamino substitué, guanidino, imidazolyle, indoyle, alkylthio inférieur, arylthio, arylthio substitué, carboxy, carbamoyle, alcoxy inférieur-carbonyle, aryle, aryle substitué, aralcoyloxy, aralcoyloxy substitué, aralkylthio ou aralkylthio substitué, la partie aryle ou hétéroaryle dudit aryloxy, hétéroaryloxy, arylamino, arylthio, aryle, aralkyloxy ou aralkylthio substitué étant substituée par un groupe choisi parmi halo, alcoyle inférieur, hydroxy, alcoxy inférieur, amino, aminométhyle, carboxyle, cyano ou sulfamoyle; $R^2$ et $R^7$ sont identiques ou différents et sont de l'hydrogène ou un alcoyle inférieur; $R^3$ est hydrogène, alcoyle inférieur, phényl-alcoyle inférieur, aminométhylphényl-alcoyle inférieur, hydroxyphényl-alcoyle inférieur, hydroxy-alcoyle inférieur, acylamino-alcoyle inférieur, amino-alcoyle inférieur, diméthylamino-alcoyle inférieur, guanidino-alcoyle inférieur, imidazolyle-alcoyle inférieur, indolyl-alcoyle inférieur ou alcoyl inférieur-thio-alcoyle inférieur; $R^4$ et $R^5$ pris ensemble forment un groupe représenté par Q ou U, où

Q est :

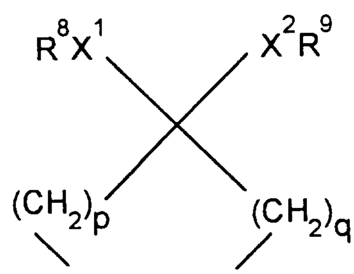

où $X^1$ et $X^2$ indépendamment l'un de l'autre sont O, S ou $CH_2$, $R^8$ et $R^9$ pris ensemble forment une liaison W, où W est un alcoylène ou un pont alcoylène substitué ayant 2 ou 3 atomes de carbone, ce pont alcoylène substitué ayant un ou plusieurs substituants choisis dans les groupes alcoyle inférieur, aryle et aryl -alcoyle inférieur, p est 0,1 ou 2, q est 0,1 ou 2, à condition que la somme de p et q doit être de 1, 2 ou 3, à condition que si p est O, alors $X^1$ et $X^2$ doivent être du méthylène.

U est

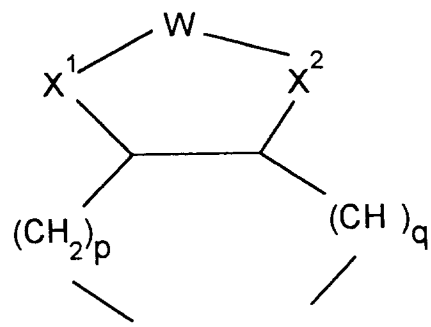

où W est un pont méthylène ou un pont méthylène substitué lorsqu'au moins l'un de $X^1$ et $X^2$ est méthylène, ou W est un pont alkylène ou alkylène substitué ayant 2 ou 3 atomes de carbone, ledit pour méthylène substitué ou ledit pour alkylène substitué ayant un ou deux substituants sélectionnés parmi les groupes alkyle inférieur, aryle et aryl alkyle inférieur (à l'exception que W peut également être un pont méthylène quand $X^1$ et $X^2$ sont de l'oxygène ou du soufre), $X^1$ et $X^2$ sont tels que définis ci-dessus, p est 0, 1 ou 2, q est 0, 1 ou 2, à condition que la somme de p et q soit 1 ou 2 et à la condition que si p est 0, $X^1$ doit être $CH_2$; à l'exclusion des composés où U est

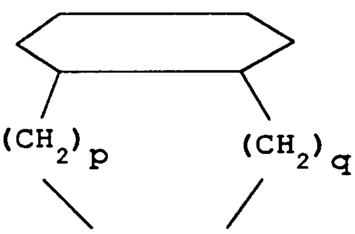

où p est zéro ou 1 et q est 1;
caractérisé en ce que le composé est préparé par un procédé approprié choisi dans ceux qui suivent (et dans les formules suivantes R, $R^1$, $R^2$, $R^3$, $R^4$, $R^5$, $R^6$ et $R^7$ sont tels que définis par la formule I, y compris la protection appropriée) :

a) pour la préparation des composés de formule I où $R^2$ est de l'hydrogène, condensation d'un composé céto (XIII) avec un dipeptide (XIV) sous réduction

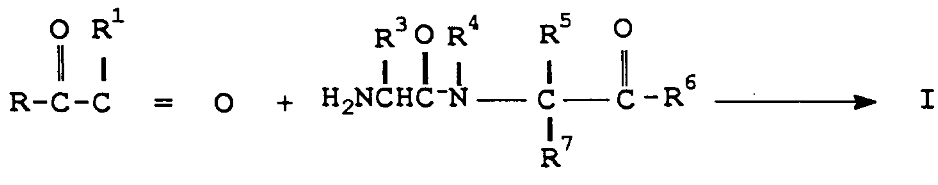

XIII XIV

b) alcoylation d'un dipeptide (XIV) au moyen d'un composé (XXII)

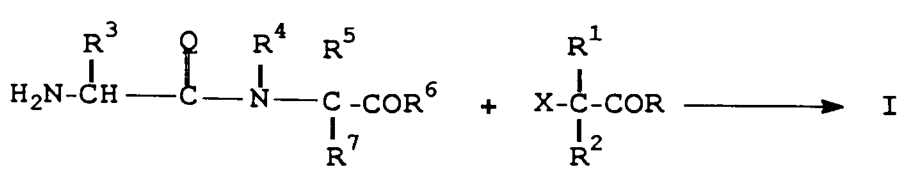

XIV XXII

où X est chloro, bromo, iodo, alcanesulfonyloxy ou arènesulfonyloxy;
c) condensation d'un composé amino (XVIII) avec un composé céto (XIX) sous réduction

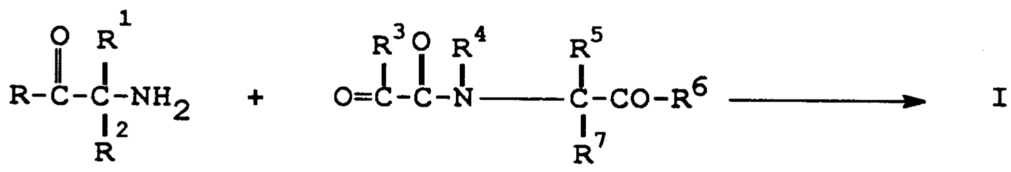

XVIII XIX

d) alcoylation d'un composé amino (XVIII) au moyen d'un composé (XXIII)

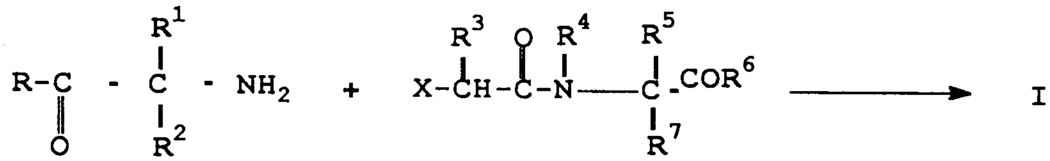

XXIII

XVIII

où X est chloro, bromo, iodo, alcanesulfonyloxy ou arènesulfonyloxy;
e) condensation d'un aminoacide (XXI) avec un aminoacide (XVII)

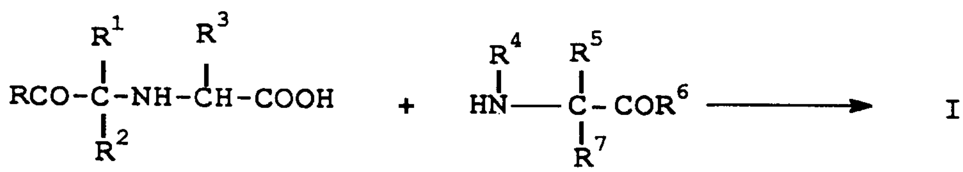

XXI XVII

suivie de l'enlèvement des groupes protecteurs si nécessaire pour donner le produit souhaité, et si on le souhaite, conversion d'un composé ainsi obtenu de formule I en un autre composé de formule I et si on le souhaite, préparation d'un sel et si on le souhaite isolement de l'isomère préféré.

**2.** Procédé selon la revendication 1, caractérisé en ce qu'un composé de formule I est préparé où $R^4$ et $R^5$ pris ensemble forment le groupe Q, où $X^1$ et $X^2$ sont méthylène, $R^8$ et $R^9$ pris ensemble forment le pont W, W étant de préférence un pont éthylène, et où p et q sont chacun de 1 ou bien où p est 0 et q est 2; ou bien où $X^1$ et $X^2$ sont S, et $R^8$ et $R^9$ pris ensemble forment un pont éthylène et p et q sont de préférence chacun 1 ou p est 1 et q est 2.

**3.** Procédé selon la revendication 1, caractérisé en ce qu'un composé de formule I est préparé où $R^4$ et $R^5$ pris ensemble forment le groupe U, où $X^1$ et $X^2$ sont méthylène et W est méthylène et p est de préférence zéro et q est de préférence 1 ou 2; ou bien où $X^1$ et $X^2$ sont méthylène et W est éthylène et où de préférence p est 0 et q est 2 ou p est 1 et q est 0; ou bien où $X^1$ ou $X^2$ sont méthylène et W est triméthylène et où de préférence p est 0 et q est 1; ou bien où $X^1$ et $X^2$ sont 0, W est méthylène et p est q sont tels que définis à la revendication 1.

**4.** Procédé selon l'une quelconque des revendications 1 à 3, caractérisé en ce qu'un composé de formule I est préparé où $R^1$ est alcoyle inférieur substitué; où le substituant est aryle non substitué ou aryle substitué en alcoyle inférieur, aralcoyloxy ou aralkylthio, $R^1$ étant de préférence alcoyle inférieur substitué, où le substituant est aralcoyloxy ou aralkylthio, de préférence benzyloxy ou benzylthio.

**5.** Procédé selon l'une quelconque des revendications 1 à 4, caractérisé en ce qu'un composé de formule I est préparé, où R et $R^6$ sont identiques ou différents et sont hydroxy, alcoxy inférieur ou aryl-alcoxy inférieur; $R^2$ et $R^7$ sont hydrogène; et $R^3$ est hydrogène, alcoyle inférieur ou phényl-alcoyle inférieur, de préférence R étant hydroxy, méthoxy ou éthoxy; $R^6$ étant hydroxy, éthoxy ou benzyloxy; $R^2$ et $R^7$ étant hydrogène; et $R^3$ étant hydrogène, méthyle ou benzyle.

**6.** Procédé selon la revendication 2, caractérisé en ce qu'un composé de formule I est préparé où $X^1$ et $X^2$ sont S, $R^8$ et $R^9$ pris ensemble forment un pont éthylène et p et q sont chacun 1, $R^1$ est phénéthyle, R et $R^6$ sont identiques ou différents et sont hydroxy ou éthoxy, $R^2$ et $R^7$ sont hydrogène et $R^3$ est hydrogène ou méthyle.

**7.** Procédé selon la revendication 6 caractérisé en ce qu'un composé est préparé, qui est l'acide 7-[N-(1-carboéthoxy-3-phénylpropyl)-(S)-alanyl-1,4-dithia-7-azaspiro [4,4] nonane-8(S)-carboxylique, l'acide 7-[N-(1-carboxy-3-phénylpropyl)-(S)-alanyl]-1,4'dithia-7-azaspiro [4,4] nonane-8(S)-carboxylique, l'acide-7-[N-(1-carboéthoxy-3-phénylpropyl)glycyl]-1,4-dithia-7-azaspiro [4,4] nonane-8(S)carboxylique, ou l'acide 7-[N-(1-carboxy-3-phénylpropyl)glycyl]-1,4-dithia-7-azaspiro [4,4] nonane-8(S)-carboxylique.

**8.** Procédé selon l'une quelconque des revendications 1 à 7, caractérisé en ce qu'un composé est préparé où les structures de la partie d'aminoacide ont la configuration S.

**9.** Procédé selon la revendication 7 ou 8, caractérisé en ce qu'un composé est préparé qui est l'acide 7-[N-(1(S)-carboéthoxy-3-phénylpropyl)-(S)-alanyl]-1,4-dithia-7-azaspiro [4,4] nonane-8(S)-carboxylique.

**10.** Procédé de préparation de compositions pharmaceutiques utiles pour le traitement de l'hypertension, caractérisé en ce qu'un composé de formule générale I tel que défini selon l'une quelconque des revendications 1 à 9 ou son sel acceptable en pharmacie, est mis sous une forme appropriée pour une administration pharmaceutique.